# EUROPEAN PATENT APPLICATION

(11) **EP 2 383 273 A1**
(43) Date of publication of application: **02.11.2011**
(21) Application number: 10733567.1
(22) Date of filing: 22.01.2010
(51) Int. Cl.: C07F 7/08, H01L 21/336, H01L 29/786, H01L 29/80, H01L 51/05, H01L 51/30, H01L 51/40

(54) **AROMATIC COMPOUND AND METHOD FOR PRODUCING SAME**

(30) Priority: 22.01.2009 JP 2009012206; 28.04.2009 JP 2009109577
(71) Applicant: Sumitomo Chemical Company, Limited, Tokyo 104-8260 (JP); Keio University, Tokyo 108-8345 (JP)
(72) Inventor: KAKIUCHI Fumitoshi, Yokohama-shi Kanagawa 223-8522 (JP); TERAI Hiroki, Tsukuba-shi Ibaraki 305-0005 (JP)
(74) Representative: Duckworth, Timothy John
(86) International application number: PCT/JP2010/050822
(87) International publication number: WO 2010/084960

(57) **Abstract**

An aromatic compound represented by the following formula (1). 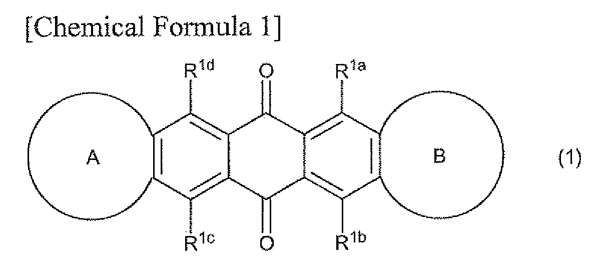 [In the formula, ring A and ring B each represent a benzene ring, an aromatic fused ring composed of 2-4 rings, a heteroaromatic ring or a heterocyclic aromatic fused ring composed of 2-4 rings, R^{1a} represents a group -CHR^{2a}-CHR^{2b}R^{2c}, and R^{1b}, R^{1c} and R^{1d} each represent hydrogen, aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each represent hydrogen, alkyl, aryl or a substituted silyl group, and R^{2a} and R^{2b} may be bonded together to form a ring. R^{2d}, R^{2e} and R^{2f} each represent hydrogen, alkyl, aryl or a substituted silyl group, and R^{2d} and R^{2e} may be bonded together to form a ring.]

## Description

### Technical Field

The present invention relates to aromatic compounds and a method for producing them and to a method for producing polyacene compounds, as well as to an organic thin-film comprising an aromatic compound or polyacene compound and an organic thin-film transistor comprising it.

### Background Art

Polyacenes exhibit high carrier mobility as materials for organic transistor elements. However, because polyacenes have low solubility in solvents, it is difficult to form films by coating methods such as spin coating or ink-jet methods when they are utilized as materials for organic transistor elements, and the usual methods for film formation involve vapor deposition that requires vacuum equipment. Film formation by coating methods can increase area size and lower cost compared to vapor deposition methods, and it is therefore desirable to develop polyacenes with introduced alkyl groups, which have high solubility in solvents.

A known example of a polyacene with alkyl groups is di-*tert*-butylpentacene (Patent document 1). The compound di-*tert*-butylpentacene is synthesized by first obtaining a pentacenequinone compound (aromatic quinone compound) by condensation reaction between 4-*tert*-butylphthalaldehyde and 1,4-cyclohexanedione, and then reducing the pentacenequinone compound in the presence of aluminum-tri-*sec*-butoxide.

### Citation List

### Patent Literature

[Patent document 1] Japanese Unexamined Patent Application Publication No. 2007-335772

### Summary of Invention

### Technical Problem

However, polyacenes obtained by the aforementioned method for producing polyacenes via aromatic quinone compounds have had alkyl groups as substituents only on the terminal benzene ring. This is because the conventional synthesis methods do not allow introduction of substituents such as alkyl groups at the peri-position with respect to the carbonyl group of the aromatic quinone compound (the site of substitution on the benzene ring adjacent to the quinone skeleton), and it has therefore been essential to accomplish synthesis with an aromatic quinone compound having an alkyl group introduced only at the terminal benzene ring.

Conventional polyacenes having alkyl groups introduced at the terminal benzene ring have exhibited a certain degree of solubility for solvents, but in order to meet recent demands for even larger areas and homogeneity of films necessary for them, even further increased solubility is being required for polyacenes.

It is an object of the present invention to satisfy this demand by providing polyacene compounds that can exhibit sufficient carrier mobility and have excellent solubility in solvents, as well as aromatic compounds that are useful as starting compounds to obtain the polyacene compounds, and a method for producing the same. It is another object of the invention to provide a method for producing polyacene compounds using the aromatic compounds, an organic thin-film comprising the aromatic compounds or polyacene compounds, and an organic thin-film transistor comprising the same.

### Solution to Problem

In order to achieve the objects stated above, the invention provides aromatic compounds represented by the following formula (1). 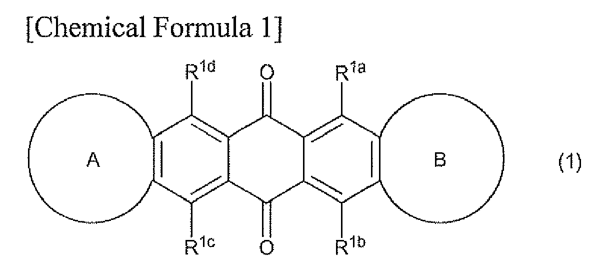 [In the formula, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups.]

Since the aromatic compounds of the invention have an alkyl group represented by -CHR^{2a}-CHR^{2b}R^{2c} substituting on the benzene ring adjacent to the quinone skeleton, when they are used as starting materials for synthesis of polyacene compounds they yield polyacene compounds having alkyl groups introduced on interior benzene rings. Such polyacene compounds can exhibit excellent carrier mobility while also having high solubility in solvents. Furthermore, the aromatic compounds of the invention themselves can also exhibit excellent carrier mobility while having excellent solubility.

An aromatic compound of the invention also has at least 2 aryl groups or alkyl groups represented by -CHR^{2d}-CHR^{2e}R^{2f} substituting on the benzene ring adjacent to the quinone skeleton, in addition to the aforementioned alkyl group. An aromatic compound thus having multiple substituents including an alkyl group on the benzene ring adjacent to the quinone skeleton can exhibit very excellent solubility, as can a polyacene compound obtained using it. Furthermore, as mentioned above, it has been difficult in the past to introduce substituents at the peri-position of aromatic quinone compounds with respect to the carbonyl group, but the aromatic compounds of the invention having the structure prescribed above can be satisfactorily obtained by the production method of the invention described hereunder.

In such aromatic compounds, preferably ring A and ring B are each independently an optionally substituted benzene ring or an optionally substituted aromatic fused ring composed of 2-4 rings.

Such an aromatic compound is more preferably a compound represented by the following formula (2). 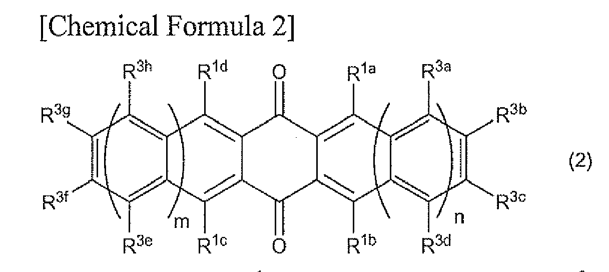 [In the formula, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. The denotations m and n each independently represent an integer of 1-3. R^{3a}, R^{3b}, R^{3c}, R^{3a}, R^{3e}, R^{3f}, R^{3g} and R^{3h} each independently represent hydrogen, optionally substituted alkyl, optionally substituted alkoxy or optionally substituted aryl. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups.]

In the aromatic compounds mentioned above, R^{1b}, R^{1c} and R^{1d} are preferably each independently a group represented by -CHR^{2d}-CHR^{2e}R^{2f}.

Also, in the aromatic compounds mentioned above, preferably m = n and more preferably m = n = 1.

An aromatic compound of the invention as described above is more preferably a compound represented by the following formula (3). 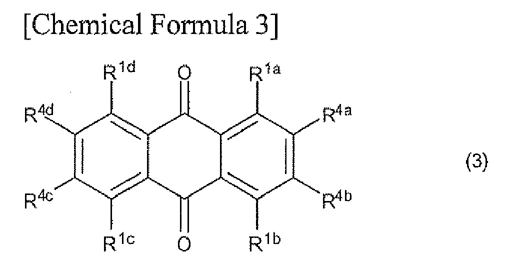 [In the formula, R^{1a} and R^{1c} are each a group represented by -CHR^{2a}-CHR^{2b}R^{2c} and are the same group, R^{1b} and R^{1d} are each an optionally substituted aryl group or a group represented by -CHR^{2d}-CHR^{2e}R^{2f} and are the same group, and R^{4a} and R^{4b}, and R^{4c} and R^{4d}, are bonded together to form ring B and ring A, in which R^{4a} and R^{4c} are the same group and R^{4b} and R^{4d} are the same group. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring.]

Ring A and ring B in the aromatic compound of the invention preferably each have a structure represented by the following formula (12). 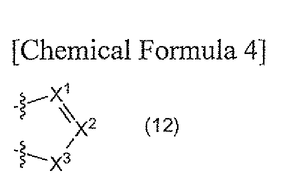 [In the formula, X¹ and X² each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group.]

The invention further provides a method for producing an aromatic compound represented by the following formula (6), which method comprises a step of addition reaction of a compound represented by the following formula (5) with a compound represented by the following formula (4), in the presence of a transition metal complex. 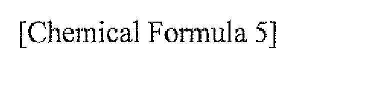 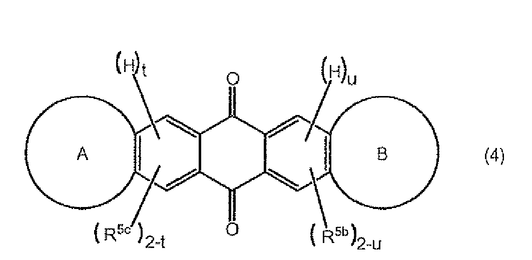 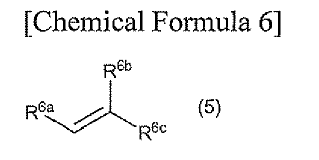 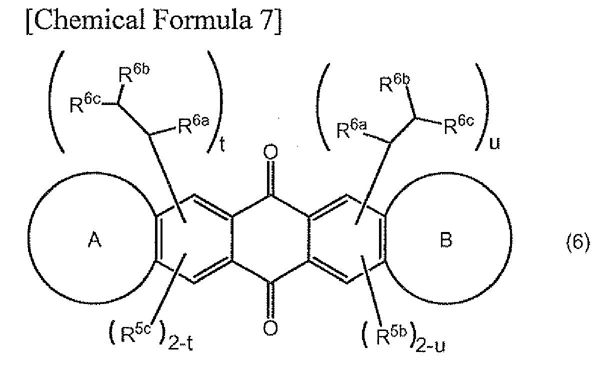 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{5b} and R^{5c} each independently represent hydrogen, optionally substituted aryl or a group -CHR^{8a}-CHR^{8b}R^{8c}_{.} R^{6a}, R^{6b} and R^{6c} each independently represent hydrogen, optionally substituted alkyl, optionally substituted aryl or a substituted silyl group, and R^{6a} and R^{6b} may be bonded together to form a ring. R^{8a}, R^{8b} and R^{8c} each independently represent hydrogen, optionally substituted alkyl, optionally substituted aryl or a substituted silyl group, and R^{8a} and R^{8b} may be bonded together to form a ring. The denotations t and u each independently represent an integer of 0-2, and are values such that t+u ≥ 1 is satisfied. In the case of two R^{5c} groups, the two R^{5c} groups may be the same or different, and in the case of two R^{5b} groups the two R^{5b} groups may be the same or different.]

In this production method of the invention, it is possible to easily accomplish addition reaction of the compound represented by formula (5) onto the carbon-hydrogen bond at the peri-position with respect to the carbonyl group in the compound represented by formula (4) (aromatic quinone compound), and as a result, it is possible to satisfactorily obtain an aromatic compound of the invention having an alkyl group derived from the compound represented by formula (5) introduced at the peri-position with respect to the carbonyl group.

In this method for producing an aromatic compound, preferably ring A and ring B are each independently an optionally substituted benzene ring or an optionally substituted aromatic fused ring composed of 2-4 rings.

In the method for producing an aromatic compound, preferably at least two of R^{5b} and R^{5c} are not hydrogen atoms.

Also the transition metal complex in the method for producing an aromatic compound is preferably a complex containing a transition metal of Groups 8-10 of the Periodic Table, more preferably a ruthenium complex, and even more preferably RuH₂(PPh₃)₄ or RuH₂(CO)(PPh₃)₃.

The invention further provides a polyacene compound represented by the following formula (15). 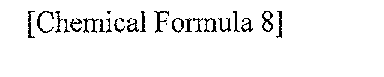 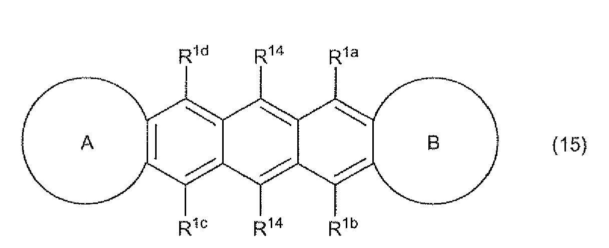 [In the formula, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2c}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups. R¹⁴ represents hydrogen or an optionally substituted alkyl, optionally substituted aryl, optionally substituted heteroaryl, optionally substituted alkenyl or optionally substituted alkynyl group. When multiple R¹⁴ groups are present, they may be the same or different.]

In the polyacene compound, R^{1b}, R^{1c} and R^{1d} are preferably each independently a group represented by -CHR^{2d}-CHR^{2e}R^{2f}.

Also in the polyacene compound, preferably R^{1a}, R^{1b}, R^{1c} and R^{1d} are the same groups and all of the substituents on ring A and ring B are the same groups.

The invention further provides a polyacene compound represented by the following formula (13). 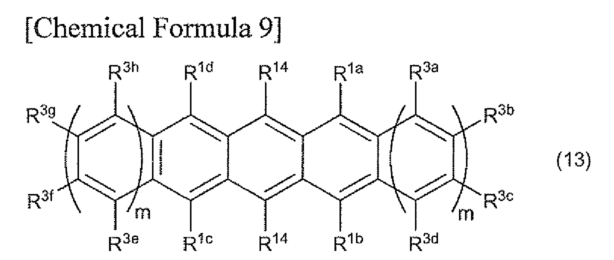 [In the formula, R^{1a} represents a group -CHR^{2a}-CHR^{2b}R^{2c}, and R^{1b}, R^{1c} and R^{1d} each independently represent hydrogen, optionally substituted aryl or a group -CHER^{2d}-CHR^{2e}R^{2f}. This is with the proviso that at least 2 of R^{1b}, R^{1c} and R^{1d} are not hydrogen. The denotation m represents an integer of 1-3. R^{3a}, R^{3b}, R^{3c}, R^{3d}, R^{3e}, R^{3f}, R^{3g} and R^{3h} each independently represent hydrogen, optionally substituted alkyl, optionally substituted alkoxy or optionally substituted aryl. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups. R¹⁴ represents hydrogen or an optionally substituted alkyl, optionally substituted aryl, optionally substituted heteroaryl, optionally substituted alkenyl or optionally substituted alkynyl group. When multiple R¹⁴ groups are present, they may be the same or different.]

In the polyacene compound, preferably m = 1.

Preferred as the polyacene compounds represented by formula (15) are polyacene compounds wherein ring A and ring B have structures represented by the following formula (12). 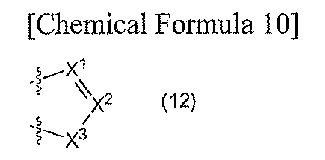 [In the formula, X¹ and X² each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by -X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group.]

In the polyacene compound, X³ is preferably a nitrogen atom, oxygen atom, sulfur atom or selenium atom, X³ is a sulfur atom, and more preferably X¹ and X² are carbon atoms.

The invention still further provides a method for producing a polyacene compound represented by the following formula (7), comprising a step of reducing an aromatic compound represented by the following formula (1). 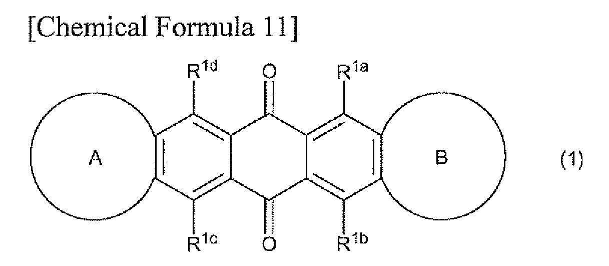 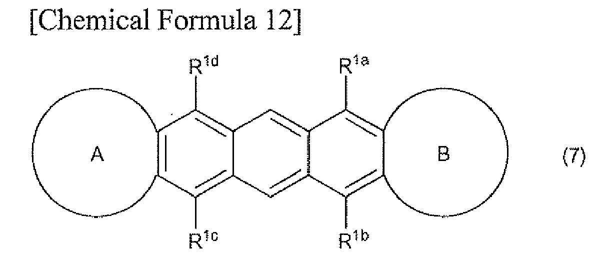 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2c}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups.]

In this method for producing a polyacene compound, preferably ring A and ring B are each independently an optionally substituted benzene ring or an optionally substituted aromatic fused ring composed of 2-4 rings.

The invention still further provides a method for producing a polyacene compound represented by the following formula (8), comprising a step of reducing a diol compound obtained by reaction between an aromatic compound represented by the following formula (1) and an organometallic compound. 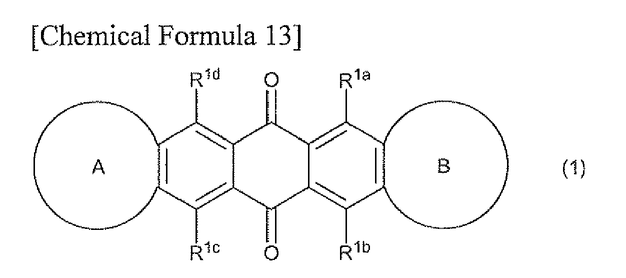 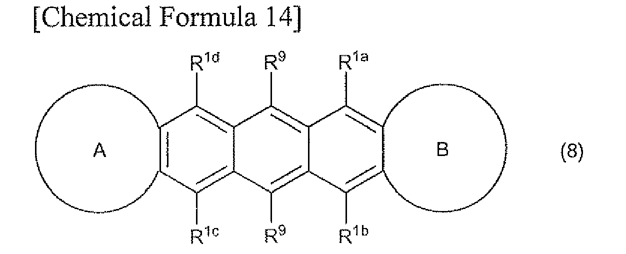 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups. R⁹ represents an optionally substituted alkyl, optionally substituted aryl, optionally substituted heteroaryl, optionally substituted alkenyl or optionally substituted alkynyl group. When multiple R⁹ groups are present, they may be the same or different.]

In this method for producing a polyacene compound, preferably ring A and ring B are each independently an optionally substituted benzene ring or an optionally substituted aromatic fused ring composed of 2-4 rings.

The invention still further provides an organic thin-film comprising an aromatic compound of the invention. The invention yet further provides an organic thin-film comprising a polyacene compound produced by the method for producing a polyacene compound of the invention. The organic thin-film of the invention can exhibit excellent carrier mobility because it comprises an aromatic compound of the invention or a polyacene compound obtained using it. Such an organic thin-film can be produced by a coating method because the aromatic compound of the invention or the polyacene compound obtained using it has high solubility in solvents, and this not only facilitates enlarged areas but also vastly reduces cost for production.

In addition, the invention provides an organic thin-film transistor comprising a source electrode and drain electrode, an organic semiconductor layer serving as a current channel between the electrodes and a gate electrode that controls the level of current flowing through the current channel, wherein the organic semiconductor layer comprises the aforementioned organic thin-film. Such an organic thin-film transistor of the invention, comprising an organic thin-film of the invention having high carrier mobility, can thereby exhibit excellent properties as a transistor.

### Advantageous Effects of Invention

According to the invention it is possible to provide aromatic compounds that are useful as starting compounds for obtaining polyacene compounds that can exhibit sufficient carrier mobility and have excellent solubility for solvents, as well as a method for producing the same. According to the invention it is also possible to provide a method for producing polyacene compounds using the aromatic compounds, an organic thin-film comprising the aromatic compounds or polyacene compounds, and an organic thin-film transistor comprising the same.

### Brief Description of Drawings

Fig. 1 is a schematic cross-sectional view of an organic thin-film transistor according to a first embodiment.
Fig. 2 is a schematic cross-sectional view of an organic thin-film transistor according to a second embodiment.
Fig. 3 is a schematic cross-sectional view of an organic thin-film transistor according to a third embodiment.
Fig. 4 is a schematic cross-sectional view of an organic thin-film transistor according to a fourth embodiment.
Fig. 5 is a schematic cross-sectional view of an organic thin-film transistor according to a fifth embodiment.
Fig. 6 is a schematic cross-sectional view of an organic thin-film transistor according to a sixth embodiment.
Fig. 7 is a schematic cross-sectional view of an organic thin-film transistor according to a seventh embodiment.
Fig. 8 is a schematic cross-sectional view of an organic thin-film transistor fabricated in the examples.

### Description of Embodiments

Preferred embodiments of the invention will now be explained in detail, with reference to the accompanying drawings as necessary. In the following explanation, methyl groups may be indicated by "Me", and ethyl groups may be indicated by "Et". Also, throughout the explanation of the drawings, corresponding elements will be referred to by like reference numerals and will be explained only once.

### [Aromatic compounds]

Aromatic compounds according to a preferred embodiment will be explained first. An aromatic compound according to this embodiment is a compound represented by formula (1) above.

In formula (1), ring A and ring B are each independently an optionally substituted benzene ring, an optionally substituted aromatic fused ring composed of 2-4 rings, an optionally substituted heteroaromatic ring or an optionally substituted heterocyclic aromatic fused ring composed of 2-4 rings. Examples of the aromatic fused rings include naphthalene, anthracene, phenanthrene, pyrene and tetracene rings. Heteroaromatic rings include furan, thiophene and selenophene rings. Heterocyclic aromatic fused rings include benzofuran, benzothiophene, benzoselenophene and thienothiophene rings.

The benzene ring, aromatic fused ring composed of 2-4 rings, optionally substituted heteroaromatic ring or optionally substituted heterocyclic aromatic fused ring composed of 2-4 rings is optionally substituted with a halogen atom, alkyl, alkoxy, aryl or the like. Alkyl groups include straight-chain, branched and cyclic groups. The aryl groups may have alkyl, alkoxy or aryl groups, or halogen atoms, as substituents. These groups may have some or all of their hydrogens replaced with halogen atoms (especially fluorine atoms).

Preferred alkyl groups are C1-20 alkyl groups (abbreviated as "C₁-C₂₀ alkyl groups", same hereunder). Such alkyl groups include methyl, ethyl, *n*-propyl, *iso*-propyl, *n*-butyl, *iso*-butyl, *sec*-butyl, *tert*-butyl, pentyl, hexyl, heptyl, octyl, 2-ethylhexyl, nonyl, decyl, lauryl, cyclopropyl, cyclopentyl, cyclohexyl, cycloheptyl, cyclooctyl, cyclononyl and cyclododecyl. C₁-C₁₂ alkyl groups are preferred, and straight-chain alkyl groups are more preferred, from the viewpoint of both achieving satisfactory solubility in solvents and maintaining satisfactory packing between molecules. Also, some or all of the hydrogens of the alkyl groups may be replaced with halogen atoms (especially fluorine atoms). Such alkyl groups include trifluoromethyl and perfluorohexyl.

Preferred alkoxy groups are those wherein the alkyl groups are C₁-C₂₀ alkyl groups. Examples of C₁-C₂₀ alkyl groups include the same ones mentioned above. Also, some or all of the hydrogens of the alkoxy groups may be replaced with halogen atoms (especially fluorine atoms).

Preferred aryl groups are C₆-C₆₀ aryl groups. Such aryl groups include phenyl, phenyl with C₁-C₁₂ alkyl groups, phenyl with C₁-C₁₂ alkoxy groups, 1-naphthyl, 2-naphthyl, 1-anthracenyl, 2-anthracenyl, 9-anthracenyl and the like. Preferred among these are C₆-C₁₄ aryl groups, with C₆-C₁₀ aryl groups being more preferred. Also, some or all of the hydrogens of the aryl groups may be replaced with halogen atoms (especially fluorine atoms).

In formula (1), R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. However, at least two among R^{1b}, R^{1c} and R^{1d} are not hydrogen atoms, and are optionally substituted aryl groups or groups represented by -CHR^{2d}-CHR^{2e}R^{2f}. R^{1b}, R^{1c} and R^{1d} are preferably groups represented by -CHR^{2d}-CHR^{2e}R^{2f}.

The optionally substituted aryl group is preferably a C₆-C₆₀ aryl group. Examples include phenyl, 1-naphthyl, 2-naphthyl, 1-anthracenyl, 2-anthracenyl and 9-anthracenyl. Preferred among these are C₆-C₁₄ aryl groups, with C₆-C₁₀ aryl groups being more preferred. The aryl groups may have alkyl, alkoxy or aryl groups, or halogen atoms, as substituents. Examples of alkyl, alkoxy and aryl groups include the same ones mentioned above.

R^{2a}, R^{2b} and R^{2c} each independently represent hydrogen, optionally substituted alkyl, optionally substituted aryl or a substituted silyl group, and R^{2a} and R^{2b} may be bonded together to form a ring.

R^{2d}, R^{2e} and R^{2f} each independently represent hydrogen, optionally substituted alkyl, optionally substituted aryl or a substituted silyl group, and R^{2d} and R^{2e} may be bonded together to form a ring.

The optionally substituted alkyl group is preferably a C₁-C₂₀ alkyl group. Such alkyl groups include methyl, ethyl, *n*-propyl, *iso*-propyl, *n*-butyl, *iso*-butyl, *sec*-butyl, *tert*-butyl, pentyl, hexyl, heptyl, octyl, 2-ethylhexyl, nonyl, decyl, lauryl, cyclopropyl, cyclopentyl, cyclohexyl, cycloheptyl, cyclooctyl, cyclononyl and cyclododecyl. C₁-C₁₂ alkyl groups are preferred, and straight-chain alkyl groups are especially preferred, from the viewpoint of both achieving satisfactory solubility in solvents and maintaining satisfactory packing between molecules. The alkyl groups may have alkoxy or aryl groups, or halogen atoms, as substituents. Examples of alkoxy and aryl groups include the same ones mentioned above. The alkyl groups may have some or all of their hydrogen atoms replaced with halogen atoms (especially fluorine atoms), and such alkyl groups include trifluoromethyl and perfluorohexyl groups.

Examples of optionally substituted aryl groups include the same ones mentioned above.

Preferred substituted silyl groups include C₁-C₆₀ substituted silyl groups, with C₃-C₄₈ substituted silyl groups being more preferred. Examples of substituted silyl groups include silyl groups substituted with 1-3 groups selected from the group consisting of alkyl, alkoxy and aryl groups. Examples of such silyl groups include trimethylsilyl, triethylsilyl, tripropylsilyl, tri-i-propylsilyl, phenyldimethylsilyl, t-butyldimethylsilyl, t-butyldiphenylsilyl, triphenylsilyl, trimethoxysilyl, triethoxysilyl and dimethylethoxysilyl.

When R^{2a} and R^{2b} bond together to form a ring, examples of groups represented by -CHR^{2a}-CHR^{2b}R^{2c} include the following.

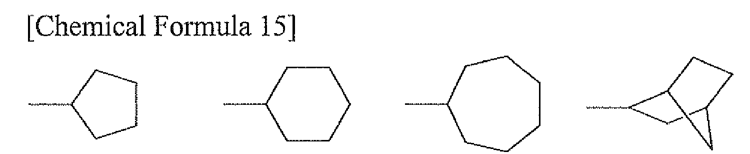

Also, when R^{2d} and R^{2e} bond together to form a ring, examples of groups represented by -CHR^{2d}-CHR^{2e}R^{2f} include the following.

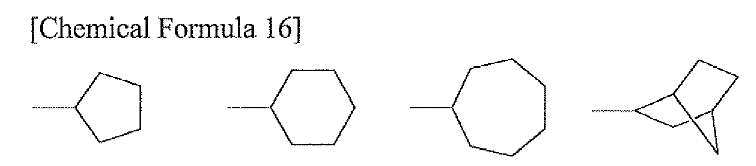

In the aromatic compound of this embodiment, preferably ring A and ring B are each independently an optionally substituted benzene ring or an optionally substituted aromatic fused ring composed of 2-4 rings, from the viewpoint of increasing the solubility in solvents.

The aromatic compound of this embodiment is more preferably a compound represented by the following formula (2), from the viewpoint or solubility in solvents.

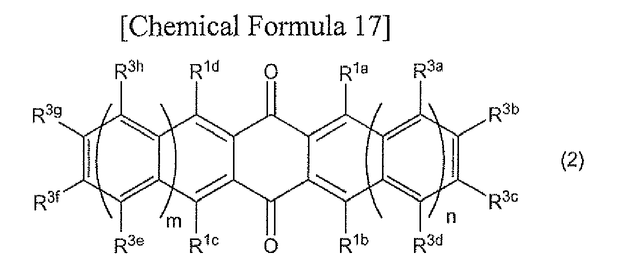

In the formula, m and n each independently represent an integer of 1-3, and R^{3a}, R^{3b}, R^{3c}, R^{3d}, R^{3e}, R^{3f}, R^{3g} and R^{3h} each independently represent hydrogen, optionally substituted alkyl, optionally substituted alkoxy or optionally substituted aryl. R^{1a}, R^{1b}, R^{1c} and R^{1d} are the same as R^{1a}, R^{1b} and R^{1c} and R^{1d} in formula (1) above. Examples of optionally substituted alkyl groups and optionally substituted aryl groups include the same ones mentioned above.

Preferred optionally substituted alkoxy groups are alkoxy groups wherein the alkyl groups are C₁-C₂₀ alkyl groups. Examples of C₁-C₂₀ alkyl groups include the same ones mentioned above. The alkoxy groups may have alkyl, alkoxy or aryl groups, or halogen atoms, as substituents. Examples of alkyl, alkoxy and aryl groups include the same ones mentioned above.

In formula (2), preferably m = n. When m = n, the aromatic compound will tend to have even more excellent packing between molecules, due to increased symmetry of the molecular structure. Most preferably, m = n = 1. A polyacene compound synthesized from the aromatic compound in which m = n = 1. will have even more excellent solubility in solvents and carrier mobility.

The aromatic compound of this embodiment preferably has a point symmetrical structure, with the center of the six-membered ring of the quinone skeleton of the aromatic compound as the reference point. A polyacene compound synthesized from an aromatic compound having a point symmetrical structure will result in even more excellent solubility in solvents and carrier mobility. From this viewpoint, the aromatic compound is preferably a compound represented by the following formula (3), for example.

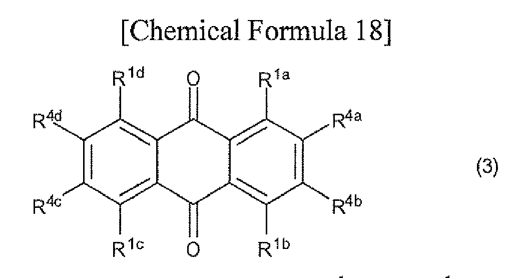

In formula (3), R^{1a} and R^{1c} are each a group represented by -CHR^{2a}-CHR^{2b}R^{2c} and are the same group, R^{1b} and R^{1d} are each an optionally substituted aryl group or a group represented by -CHR^{2d}-CHR^{2e}R^{2f} and are the same group, and R^{4a} and R^{4b}, and R^{4c} and R^{4d}, are bonded together to form ring B and ring A, in which R^{4a} and R^{4c} are the same group and R^{4b} and R^{4d} are the same group. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. Examples of the optionally substituted aryl, optionally substituted alkyl, optionally substituted aryl and substituted silyl groups are the same as those mentioned above.

Preferred as aromatic compounds of this embodiment are those in which ring A and ring B have a structure represented by the following formula (12). The structure represented by formula (12) may be vertically inverted with respect to the benzene ring to which it is bonded.

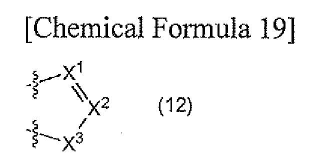

In the formula, X¹ and X² each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by -X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. Examples of optionally substituted alkyl groups, optionally substituted alkoxy groups and optionally substituted aryl groups include the same ones mentioned above.

In formula (12), more preferably X¹ and X² are carbon atoms and X³ is a sulfur atom. Such an aromatic compound is useful as a starting compound to obtain a polyacene compound with even more excellent solubility in solvents and carrier mobility.

Examples of structures represented by formula (12) include structures represented by the following formulas (001), (002), (003), (004), (005), (006), (007), (008), (009), (010), (011), (012) and (013).

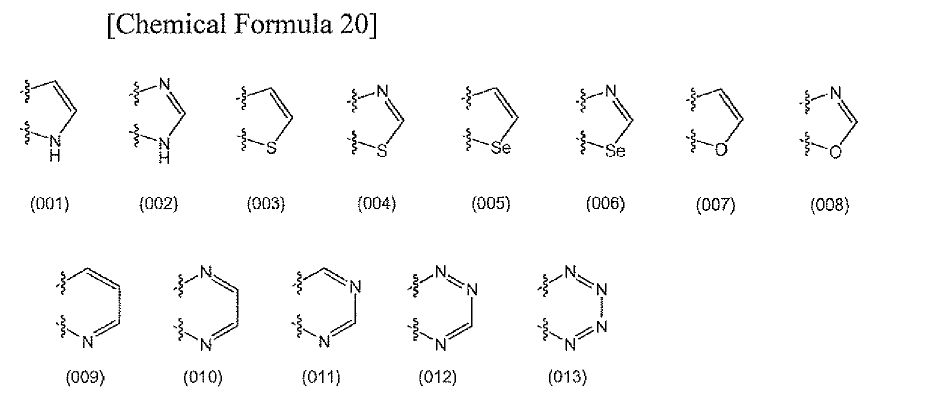

Examples of aromatic compounds of this embodiment include aromatic compounds represented by the following formulas (101), (102), (103), (104), (105), (106), (107), (108), (109), (110), (111), (112), (113), (114), (115), (116), (117), (118), (119), (120) and (121). Preferred among these are aromatic compounds represented by the following formulas (105), (106), (107), (108) and (112).

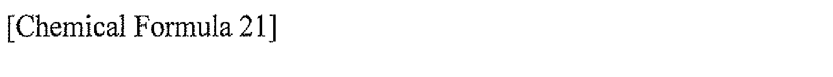

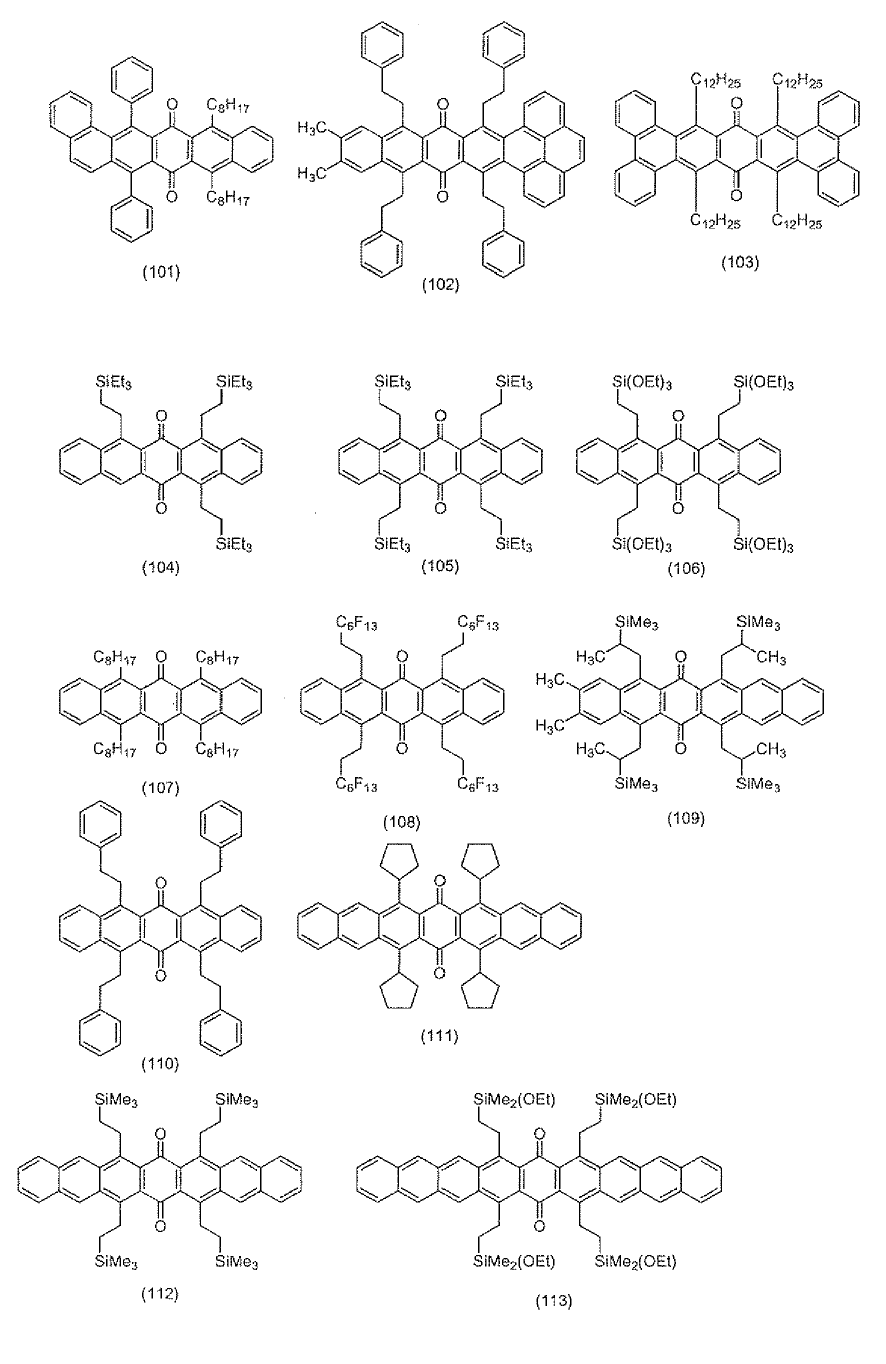

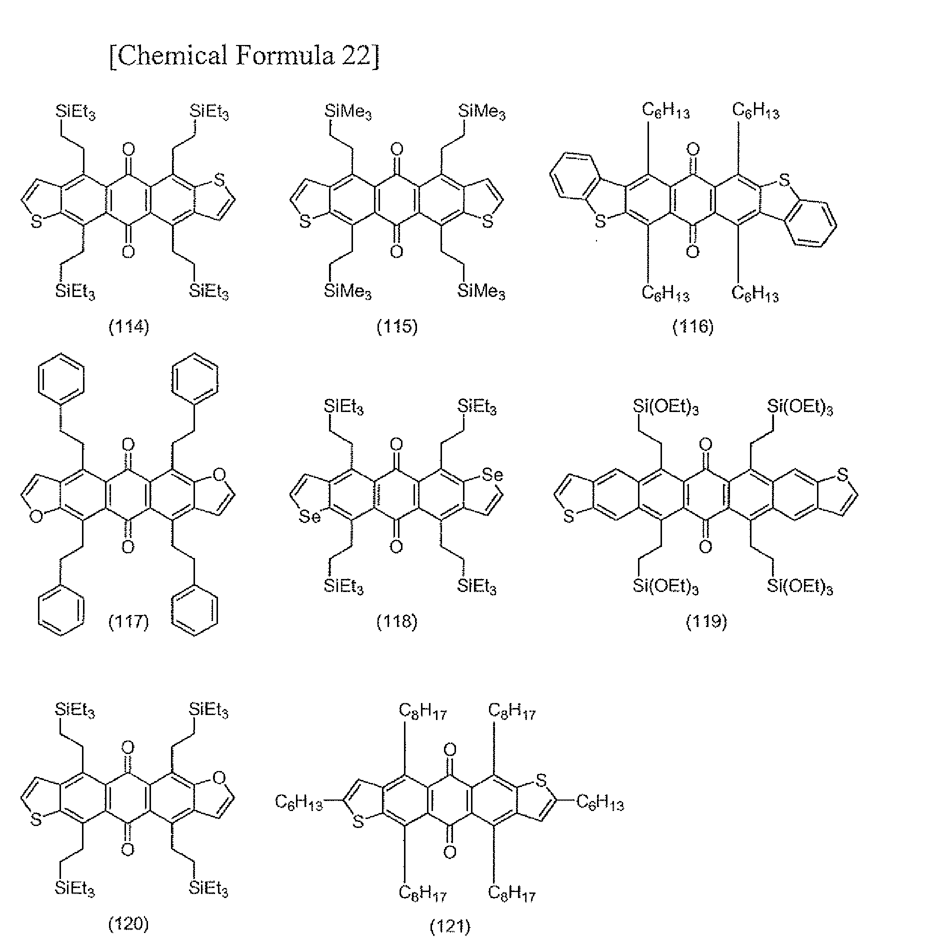

### [Method for producing aromatic compound]

The method for producing an aromatic compound according to this embodiment produces an aromatic compound represented by the following formula (6) by addition reaction of a compound represented by the following formula (5) with a compound represented by the following formula (4), in the presence of a transition metal complex. Specifically, the method for producing an aromatic compound according to this embodiment comprises a step of addition reaction of a compound represented by the following formula (5) with a compound represented by the following formula (4), in the presence of a transition metal complex.

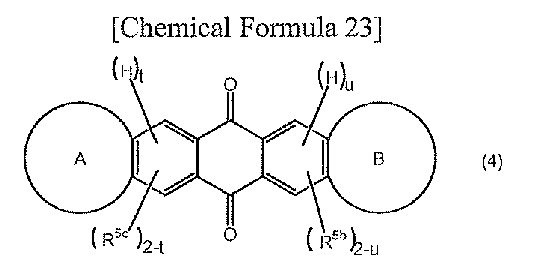

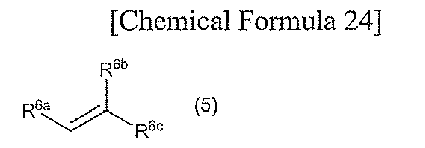

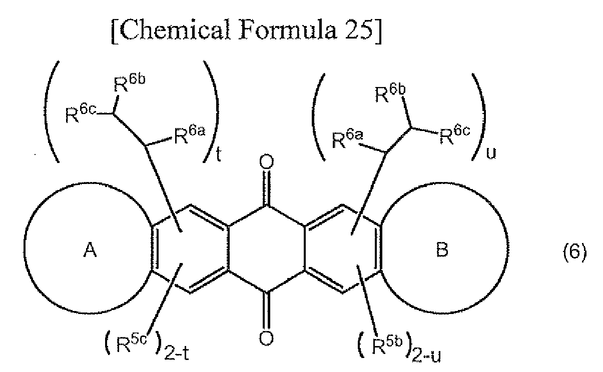

In formulas (4), (5) and (6), ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, and R^{5b} and R^{5c} each independently represent hydrogen, optionally substituted aryl or a group -CHR^{8a}-CHR^{8b}R^{8c}, Preferably at least two of R^{5b} and R^{5c} are not hydrogen atoms. Specifically, there may be a total of 0-3 R^{5b} and R^{5c} groups, and when 2 or more are present, preferably 2 or more are not hydrogen. R^{6a}, R^{6b} and R^{6c} each independently represent hydrogen, optionally substituted alkyl, optionally substituted aryl or a substituted silyl group, and R^{6a} and R^{6b} may be bonded together to form a ring. R^{8a}, R^{8b} and R^{8c} each independently represent hydrogen, optionally substituted alkyl, optionally substituted aryl or a substituted silyl group, and R^{8a} and R^{8b} may be bonded together to form a ring. The denotations t and u are each an integer of 0-2, and are values such that t+u ≥ 1 is satisfied. In the case of two R^{5c} groups, the two R^{5c} groups may be the same or different, and in the case of two R^{5b} groups the two R^{5b} groups may be the same or different.

Examples of optionally substituted benzene rings, optionally substituted aromatic fused rings composed of 2-4 rings, optionally substituted heteroaromatic rings, optionally substituted heterocyclic aromatic fused rings composed of 2-4 rings, optionally substituted aryl, optionally substituted alkyl and substituted silyl groups include the same ones mentioned above.

In the method for producing an aromatic compound according to this embodiment, a compound represented by formula (5) is added at the bond linking the carbon at the peri-position and the hydrogen at the peri-position with respect to the carbonyl group (peri-position carbon-hydrogen bond) in the compound represented by formula (4), to produce a compound represented by formula (6). If the compound represented by formula (4) has a plurality of peri-position carbon-hydrogen bonds (i.e. t+u in formula (4) is 2 or greater), addition reaction can potentially take place at any of the plurality of peri-position sites. In this case, appropriate varying of the reaction conditions can yield a product in which the compound represented by formula (5) has been added at all or some of the peri-position carbon-hydrogen bonds.

In the compound represented by formula (4), preferably ring A and ring B are each independently an optionally substituted benzene ring or an optionally substituted aromatic fused ring composed of 2-4 rings. With such a compound it is possible to obtain an aromatic compound that is suitable from the viewpoint of solubility in solvents.

The compound represented by formula (4) is preferably one such that, as a result of addition reaction, can yield an aromatic compound represented by formula (2) or (3) as the aromatic compound represented by formula (6). Such compounds include compounds represented by the following formulas (X) and (Y).

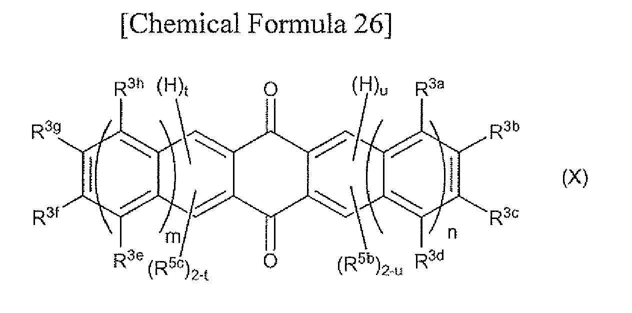

In formula (X), m, n, R^{3a}, R^{3b}, R^{3c}, R^{3d}, R^{3e}, R^{3f}, R^{3g} and R^{3h} are the same as m, n, R^{3a}, R^{3b}, R^{3c}, R^{3d}, R^{3e}, R^{3f}, R^{3g} and R^{3h} for formula (2)_{.} Also, u, t, R^{5b} and R^{5c} are the same as u, t, R^{5b} and R^{5c} in formula (4).

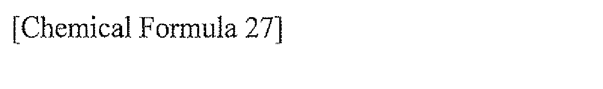

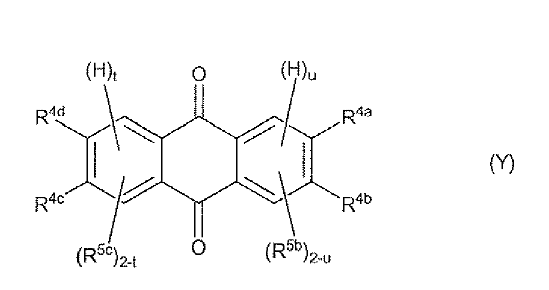

In formula (Y), R^{4a}, R^{4b}, R^{4c} and R^{4d} are the same as R^{4a}, R^{4b}, R^{4c} and R^{4d} in formula (3). Also, u, t, R^{5b} and R^{5c} are the same as u, t, R^{5b} and R^{5c} in formula (4).

Compounds represented by formula (4) include compounds represented by the following formulas (201), (202), (203), (204), (205), (206), (207), (208), (209), (210), (211), (212) and (213).

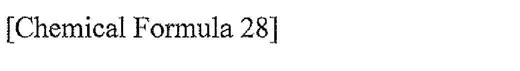

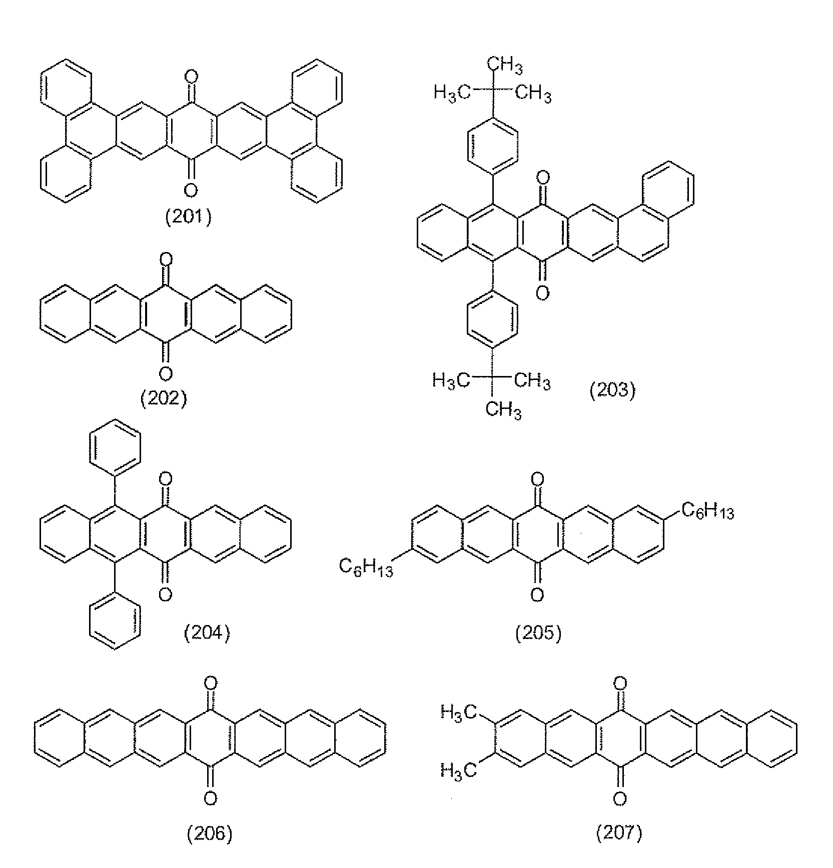

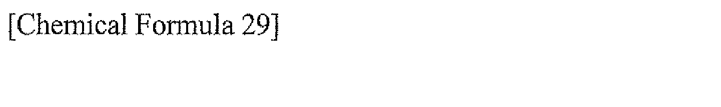

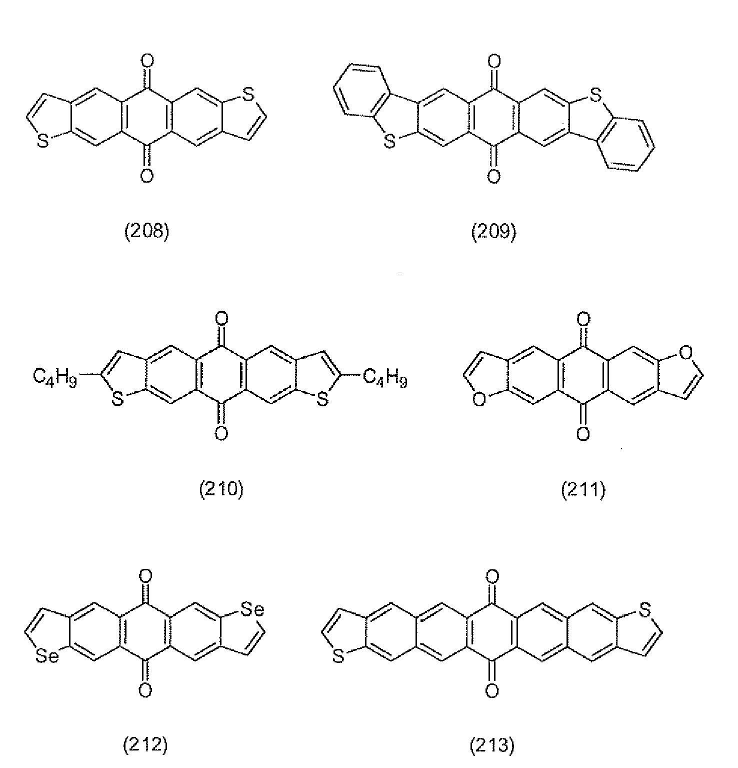

Compounds represented by formula (5) include compounds represented by the following formulas (301), (302), (303), (304), (305), (306), (307), (308), (309), (310) and (311).

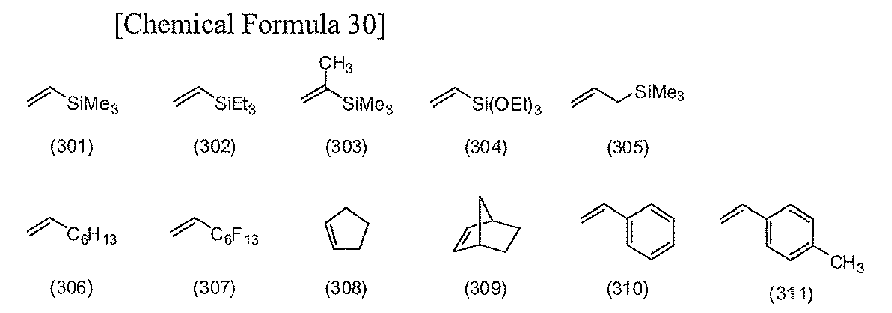

In the addition reaction of the method for producing an aromatic compound according to this embodiment, the compound represented by formula (5) is preferably used at 1-1000 mol with respect to 1 mol of the compound represented by formula (4). From the viewpoint of efficiently promoting the addition reaction, the amount of compound represented by formula (5) used is more preferably at least 1.1 mol and even more preferably at least 4 mol with respect to 1 mol of the compound represented by formula (4). From the viewpoint of increasing the reaction rate of the addition reaction, it is more preferably no greater than 100 mol and even more preferably no greater than 40 mol.

The transition metal complex used in the addition reaction is preferably a transition metal complex of groups 8-10 of the Periodic Table, more preferably it is a ruthenium complex or rhodium complex, and even more preferably it is a ruthenium complex.

Preferred transition metal complexes include RuH₂(PPh₃)₄, RuH₂(CO)(PR^{a}₃)₃ (where R^{a} represents an alkyl or aryl group, and multiple R^{a} groups may be the same or different), RuHX(CO)(PPh₃)₃ (where X represents a halogen atom), Ru(CH₂=CH₂)(PPh₃)₃ (ethylenetris(triphenylphosphine)ruthenium), RuH₂(H₂)₂(PR^{b}₃)₂ (where R^{b} represents an alkyl group, and multiple R^{b} groups may be the same or different), three-component systems of [Ru(η⁶-C₆R^{c}₆)Cl₂]₂, PR^{d}₃ and HCO₂Na (where R^{c} represents hydrogen or an alkyl group, multiple R^{c} groups may be the same or different, R^{d} represents an alkyl or aryl group, and multiple R^{d} groups may be the same or different) and (η⁵-C₅ Me₅)Rh(C₂H₃SiR^{e}₃)₂ (where R^{e} represents an alkyl group, and multiple R^{e} groups may be the same or different). More preferred among these are RuH₂(PPh₃)₄ (dihydridotetrakis(triphenylphosphine)ruthenium) and RuH₂(CO)(PPh₃)₃ (dihydridocarbonyltris(triphenylphosphine)ruthenium). These complexes may be used in combinations of two or more, and the complexes may also be used in combination with commonly known forms of ligands.

The amount of transition metal complex used is preferably 0.0001-0.5 mol and more preferably 0.01-0.2 mol with respect to 1 mol of the compound represented by formula (4).

The addition reaction will also proceed in the absence of a solvent, but a solvent may be used. The solvent used may be any solvent that is inert to the reaction, and examples include aliphatic hydrocarbon solvents such as cyclohexane and methylcyclohexane, aromatic hydrocarbon solvents such as benzene and toluene, ether-based solvents such as tetrahydrofuran and anisole, and nitrile-based solvents such as acetonitrile. Preferred among these are aromatic hydrocarbon solvents such as benzene and toluene.

The addition reaction may also be conducted in air, but it is preferably conducted in an inert gas atmosphere of nitrogen or argon. Conducting the reaction in an inert atmosphere can produce an effect of increasing the reaction yield.

During the addition reaction, the reactor that is used for the reaction may be either dried or non-dried, but preferably a dried reactor is used. Using a dried reactor can produce an effect of increasing the reaction yield.

The reaction temperature for the addition reaction is preferably 20°C-200°C and more preferably 50°C-160°C. The reaction time is preferably 0.5 minutes-200 hours and more preferably 3 minutes-50 hours. If the reaction temperature is too high or the reaction time is too long, inconveniences may result such as loss of activity of the transition metal complex serving as the catalyst. If the reaction temperature is too low or the reaction time is too short, the reaction may not proceed sufficiently and inconveniences such as low yield may result. Conducting the addition reaction so that the aforementioned conditions are satisfied can efficiently yield the target aromatic compound.

The aromatic compound of this embodiment can be produced by the following procedure, for example. First, the entire reactor is exchanged with an inert gas such as nitrogen or argon, and then the transition metal complex, the compound represented by formula (4), the compound represented by formula (5) and a solvent as necessary are placed in the container and mixed by stirring. The mixture is then heated if necessary and stirred for reaction. It may also be heated to reflux at this time.

When the compound represented by formula (5) is a gas at ordinary temperature, it is preferred to use an autoclave as the reactor. In such cases, the interior of the autoclave is exchanged with an inert gas, for example, and then the transition metal complex, the compound represented by formula (4) and the solvent if necessary, are placed therein, the gas of the compound represented by formula (5) is injected in, and heated stirring is carried out as necessary for reaction.

Upon completion of the reaction, for example, the reacted mixture may be concentrated directly, or the reacted mixture may be placed in water, an organic solvent such as toluene, ethyl acetate, diethyl ether or dichloromethane used for extraction, and the obtained organic layer subsequently concentrated to obtain the target aromatic compound represented by formula (6). The obtained aromatic compound may be purified by column chromatography, extraction, recrystallization, distillation or the like.

### [Method for producing polyacene compound]

The aromatic compound described above may be used as the starting material for a prescribed reaction to produce a polyacene compound. The method for producing a polyacene compound may be the following "polyacene compound production method (A)" or "polyacene compound production method (B)". First, in polyacene compound production method (A), preferably the aromatic compound represented by formula (1) is reduced in the presence of a reducing agent to produce a polyacene compound represented by the following formula (7). Specifically, the polyacene compound production method (A) preferably comprises a step of reducing the aromatic compound represented by formula (1) in the presence of a reducing agent.

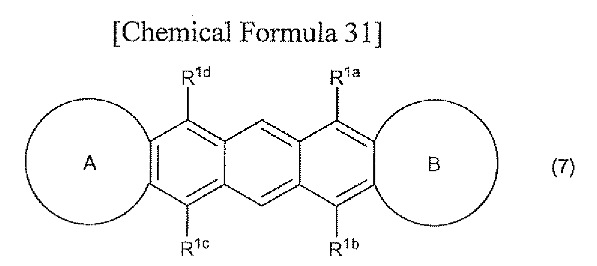

In formula (7), ring A, ring B, R^{1a}, R^{1b}, R^{1c} and R^{1d} are the same as ring A, ring B, R^{1a}, R^{1b}, R^{1c} and R^{1d} in formula (1) above.

The reducing agent used in polyacene compound production method (A) may be, for example, hydrogen, hydrogen peroxide, sulfur dioxide, hydrogen sulfide, hydrogen iodide, sodium borohydride, lithium aluminum hydride, lithium triethylborohydride, diborane, diisobutylaluminum hydride, sodium phosphinate, hydrazine, a metal such as nickel, palladium, platinum, rhodium or ruthenium, or a complex thereof. Hydrogen iodide, lithium aluminum hydride and sodium phosphinate are preferably used among these because high reactivity can be obtained.

In the polyacene compound production method (A), the reaction temperature is preferably 0-200°C and the reaction time is preferably 3 minutes- 100 hours.

In the compound represented by formula (7), preferably ring A and ring B are each independently an optionally substituted benzene ring or an optionally substituted aromatic fused ring composed of 2-4 rings, from the viewpoint of solubility in solvents. Such a polyacene compound can be obtained by using a compound represented by formula (1) wherein ring A and ring B are each independently an optionally substituted benzene ring or an optionally substituted aromatic fused ring composed of 2-4 rings.

Compounds represented by formula (7) include compounds represented by the following formulas (401), (402), (403), (404), (405), (406), (407), (408), (409), (410), (411), (412), (413), (414), (415) and (416).

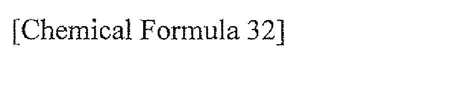

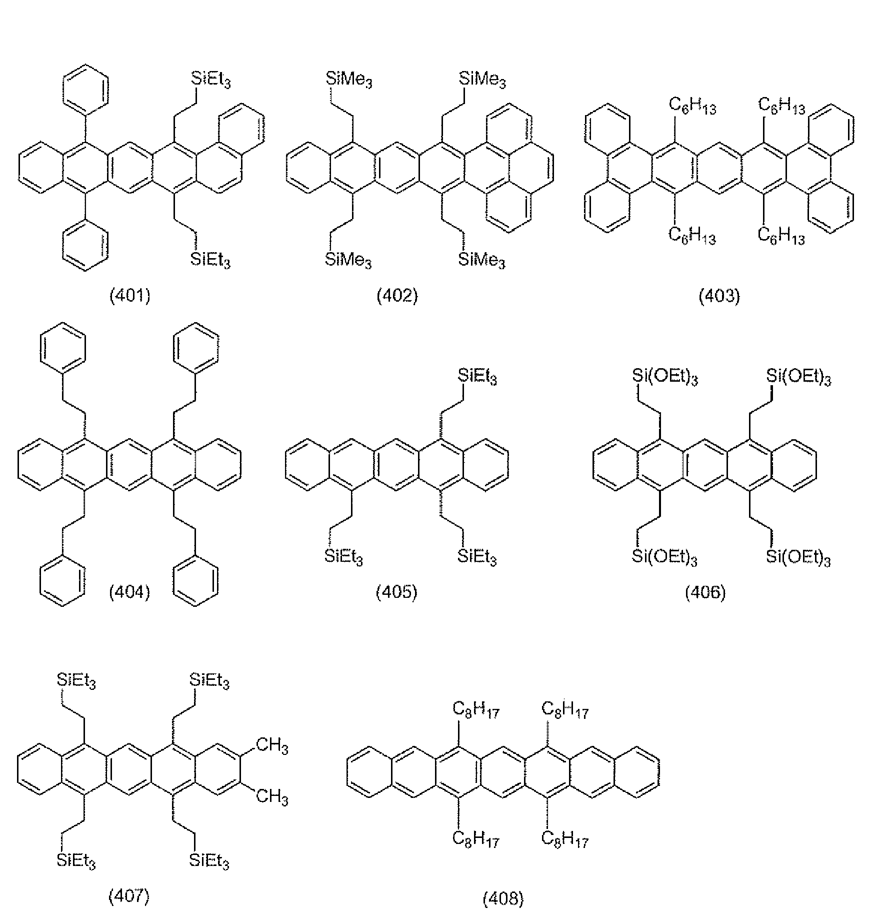

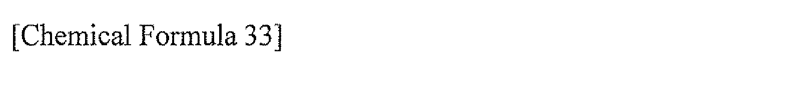

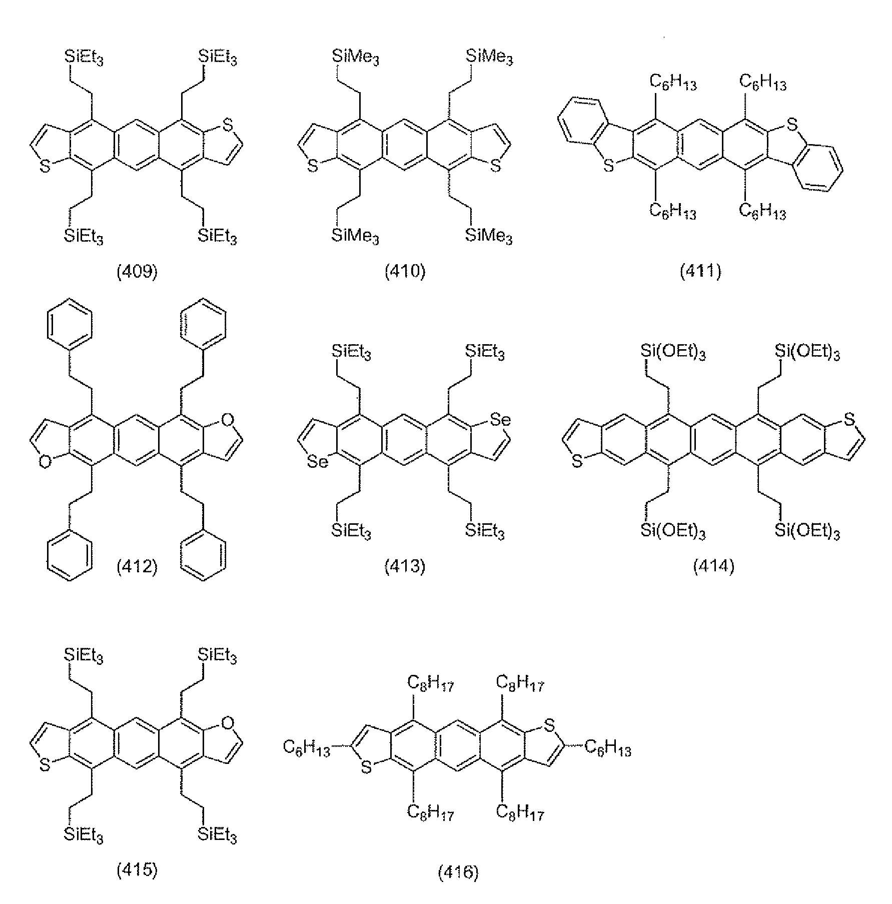

In polyacene compound production method (B), a diol compound obtained by reaction between the aromatic compound represented by formula (1) and an organometallic compound is reduced, preferably in the presence of a reducing agent, to produce a polyacene compound represented by the following formula (8). Specifically, the polyacene compound production method (B) comprises a step of reducing a diol compound obtained by reaction between an aromatic compound represented by formula (1) and an organometallic compound, preferably in the presence of a reducing agent.

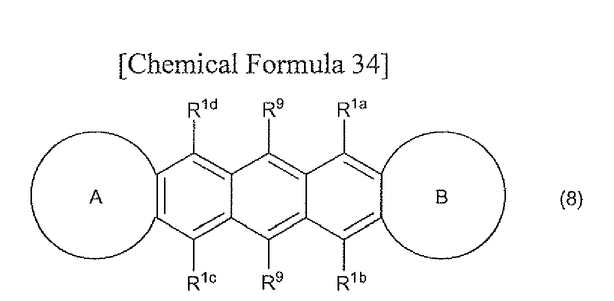

In formula (8), ring A, ring B, R^{1a}, R^{1b}, R^{1c} and R^{1d} are the same as ring A, ring B, R^{1a}, R^{1b}, R^{1c} and R^{1d} in formula (1) above. R⁹ represents an optionally substituted alkyl, optionally substituted aryl, optionally substituted heteroaryl, optionally substituted alkenyl or optionally substituted alkynyl group. Examples of optionally substituted alkyl groups and optionally substituted aryl groups include the same ones mentioned above.

The optionally substituted heteroaryl group is preferably a C₃₋C₆₀ heteroaryl group. Such heteroaryl groups include furyl, thienyl, thienylthienyl, thienothienyl, pyrrolyl, pyridyl, bipyridyl, C₁-C₁₂ alkylthienyl and C₁-C₁₂ alkylthienothienyl. C₃-C₁₂ heteroaryl groups are more preferred among these. The heteroaryl groups may have alkyl, alkoxy or aryl groups, or halogen atoms, as substituents. Examples of alkyl, alkoxy and aryl groups include the same ones mentioned above.

Optionally substituted alkenyl groups include groups represented by -CR¹¹=CR¹²-R¹³. Here, R¹¹, R¹² and R¹³ may each independently be hydrogen, or the aforementioned optionally substituted alkyl, optionally substituted aryl, optionally substituted heteroaryl or substituted silyl groups.

Optionally substituted alkynyl groups include groups represented by -C≡C-R¹⁰. Here, R¹⁰ may be the aforementioned optionally substituted alkyl, optionally substituted aryl, optionally substituted heteroaryl or substituted silyl groups.

Organometallic compounds to be used in polyacene compound production method (B) include organometallic compounds represented by R⁹-MXₙ. R⁹ in this case is the same as R⁹ in formula (8) above, M represents a metal atom, X represents a halogen atom and n represents an integer of 0 or greater. The metal atom represented by M is preferably lithium or magnesium. Specifically preferred organometallic compounds are organic lithium compounds and organic magnesium compounds.

The reducing agent used for polyacene compound production method (B) may be any publicly known reducing agent, but from the viewpoint of reactivity it is preferred to use tin(II) chloride, tin(II) chloride and hydrochloric acid.

In polyacene compound production method (B), as mentioned above, first the organometallic compound is reacted with the carbonyl group of the aromatic compound represented by formula (1) in the first stage, and a diol compound is produced. An example of a reaction scheme for the first stage is shown as formula (10) below.

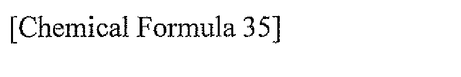

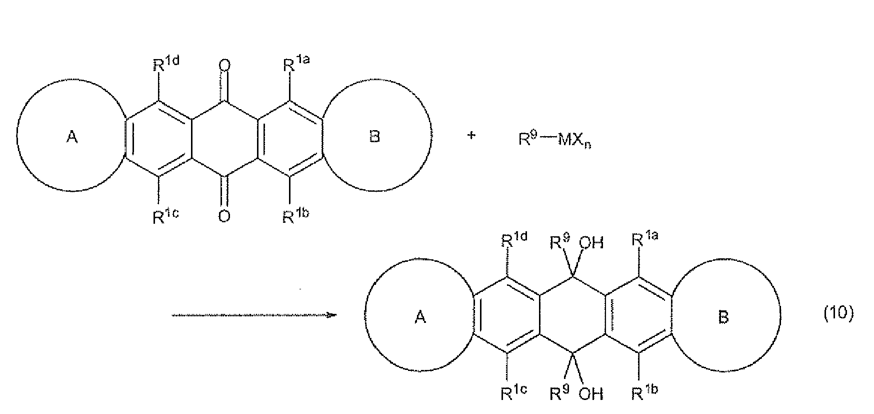

The reaction conditions for the first stage are preferably a reaction temperature of between -78 and 100°C and a reaction time of 3 minutes-10 hours.

Next, as the second stage in polyacene compound production method (B), the diol compound obtained in the first stage is reduced in the presence of a reducing agent to produce a polyacene compound. An example of a reaction scheme for the second stage is shown as formula (11) below.

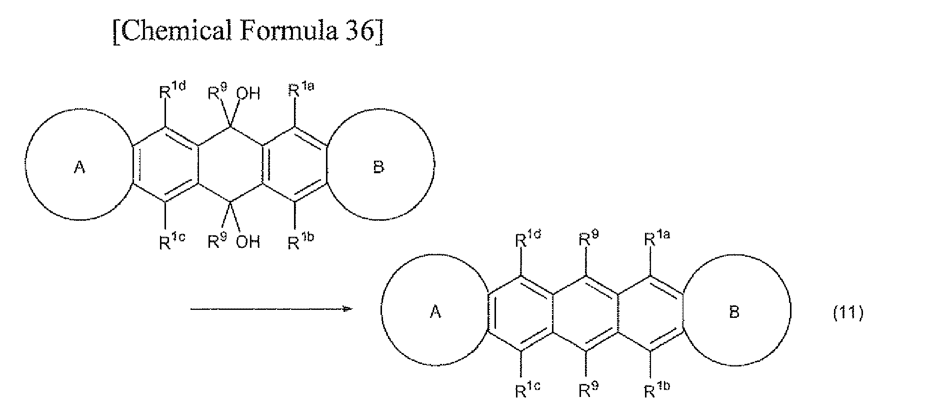

The reaction conditions for the second stage are preferably a reaction temperature of between 0 and 100°C and a reaction time of 3 minutes-10 hours.

In the compound represented by formula (8), preferably ring A and ring B are each independently an optionally substituted benzene ring or an optionally substituted aromatic fused ring composed of 2-4 rings, from the viewpoint of solubility in solvents. Such a polyacene compound can be obtained by using a compound represented by formula (1) wherein ring A and ring B are each independently an optionally substituted benzene ring or an optionally substituted aromatic fused ring composed of 2-4 rings.

Compounds represented by formula (8) include compounds represented by the following formulas (501), (502), (503), (504), (505), (506), (507), (508), (509), (510), (511), (512), (513), (514), (515) and (516).

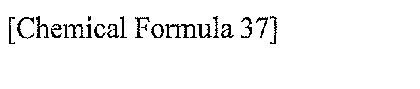

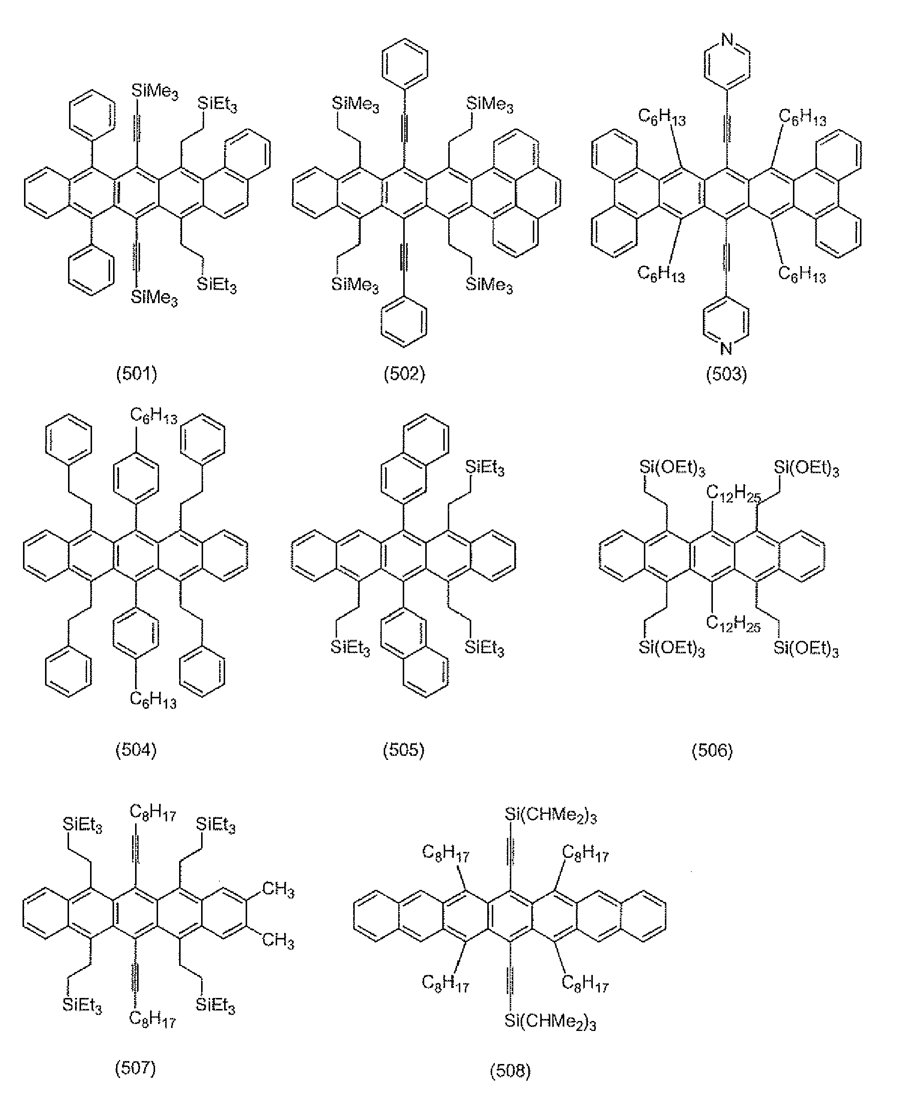

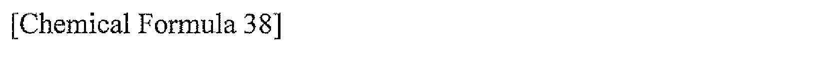

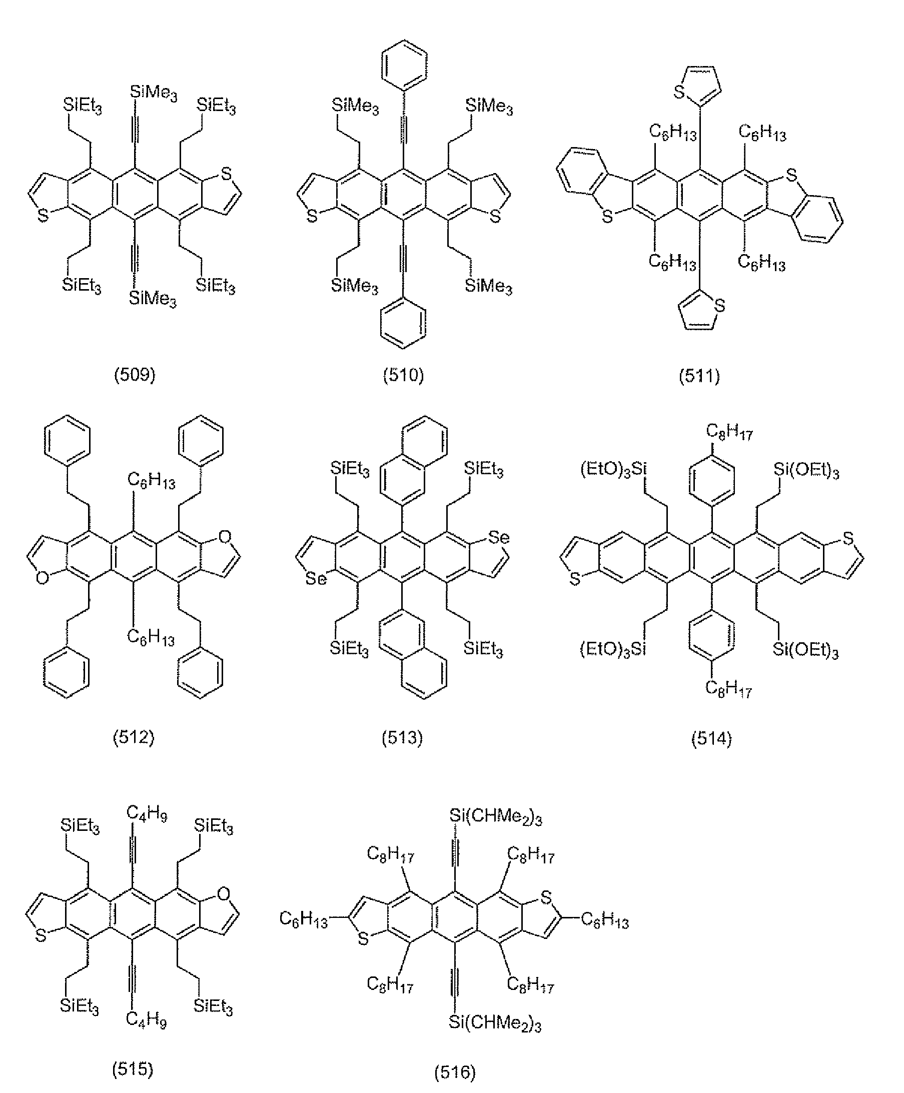

The polyacene compound obtained by production method (A) or production method (B) described above may be highly purified by a purification method such as sublimation or recrystallization. Polyacene compounds that can be produced by the method for producing a polyacene compound according to this embodiment include polyacene compounds represented by formulas (901), (902), (903), (904), (905), (906), (907), (908), (909), (910), (911), (912), (913), (914), (915), (916), (917), (918) and (919) shown below.

### [Polyacene compound]

A polyacene compound according to a preferred embodiment will now be described. A polyacene compound according to this embodiment is, for example, a polyacene compound obtained by the aforementioned polyacene compound production method (A) or production method (B), and it is a compound represented by formula (7) or formula (8) above.

The polyacene compound is preferably a polyacene compound represented by the following formula (13), from the viewpoint of increased solubility in solvents.

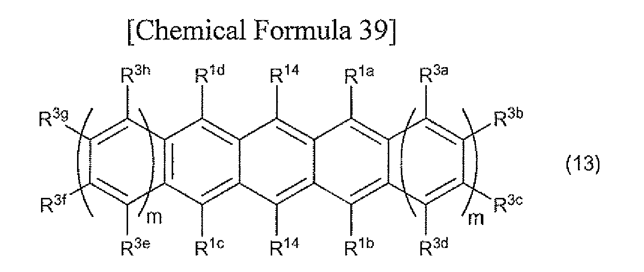

In the formula, m represents an integer of 1-3. R^{1a}, R^{1b}, R^{1c} and R^{1d} are the same as R^{1a}, R^{1b}, R^{1c} and R^{1d} in formula (1) above, and R^{3a}, R^{3b}, R^{3c}, R^{3d}, R^{3e}, R^{3f}, R^{3g} and R^{3h} are the same as R^{3a}, R^{3b}, R^{3c}, R^{3d}, R^{3e}, R^{3f}, R^{3g} and R^{3h} in formula (2) above. R¹⁴ represents hydrogen or an optionally substituted alkyl, optionally substituted aryl, optionally substituted heteroaryl, optionally substituted alkenyl or optionally substituted alkynyl group.

In the polyacene compound represented by formula (13), preferably R^{1b}, R^{1c} and R^{1d} are each independently a group represented by -CHR^{2d}-CHR^{2e}R^{2f}, from the viewpoint of increased solubility in solvents.

Also in the polyacene compound represented by formula (13), preferably R^{1a}, R^{1b}, R^{1c} and R^{1d} are the same groups, R^{3a}, R^{3d}, R^{3e} and R^{3h} are the same groups and R^{3b}, R^{3c}, R^{3f} and R^{3g} are the same groups. Such a polyacene compound will tend to have even more excellent packing between molecules due to increased symmetry of the molecular structure. Also, preferably m = 1 in the polyacene compound represented by formula (13).

The polyacene compound is preferably a polyacene compound represented by the following formula (14), from the viewpoint of even more excellent carrier mobility.

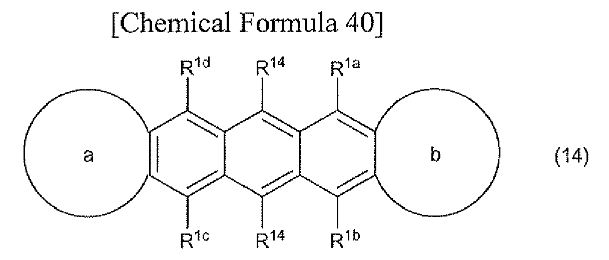

In the formula, ring a and ring b each independently represent a heteroaromatic ring having a structure represented by the following formula (12), or an aromatic fused ring composed of 2-4 rings. R^{1a}, R^{1b}, R^{1c} and R^{1d} are the same as R^{1a}, R^{1b}, R^{1c} and R^{1d} in formula (1) above, and R¹⁴ is the same as R¹⁴ in formula (13) above.

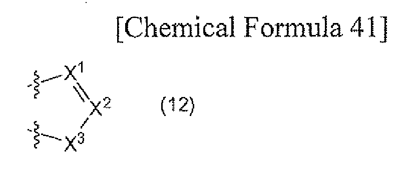

In the formula, X¹ and X² each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by -X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. Examples of optionally substituted alkyl groups, optionally substituted alkoxy groups and optionally substituted aryl groups include the same ones mentioned above.

In the polyacene compound represented by formula (14), preferably R^{1b}, R^{1c} and R^{1d} are each independently a group represented by -CHR^{2d}-CHR^{2e}R^{2f}, from the viewpoint of increased solubility in solvents.

Also in the polyacene compound represented by formula (14), preferably R^{1a}, R^{1b}, R^{1c} and R^{1d} are the same groups and ring a and ring b are the same ring. Such a polyacene compound will tend to have even more excellent packing between molecules due to increased symmetry of the molecular structure.

Preferred polyacene compounds of the polyacene compounds represented by formula (14) are those wherein X¹ and X² are carbon atoms and X³ is a sulfur atom. Such polyacene compounds will tend to have even more excellent solubility in solvents and carrier mobility.

Examples of polyacene compounds for this embodiment include polyacene compounds represented by formulas (401), (402), (403), (404), (405), (406), (407), (408), (409), (410), (411), (412), (413), (414), (415), (416), (501), (502), (503), (504), (505), (506), (507), (508), (509), (510), (511), (512), (513), (514), (515) and (516) above, as well as polyacene compounds represented by the following formulas (901), (902), (903), (904), (905), (906), (907), (908), (909), (910), (911), (912), (913), (914), (915), (916), (917), (918) and (919). Preferred among these are polyacene compounds represented by the following formulas (901), (905), (906), (907), (908), (909), (910), (911), (912), (914), (915), (916), (918) and (919).

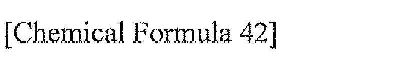

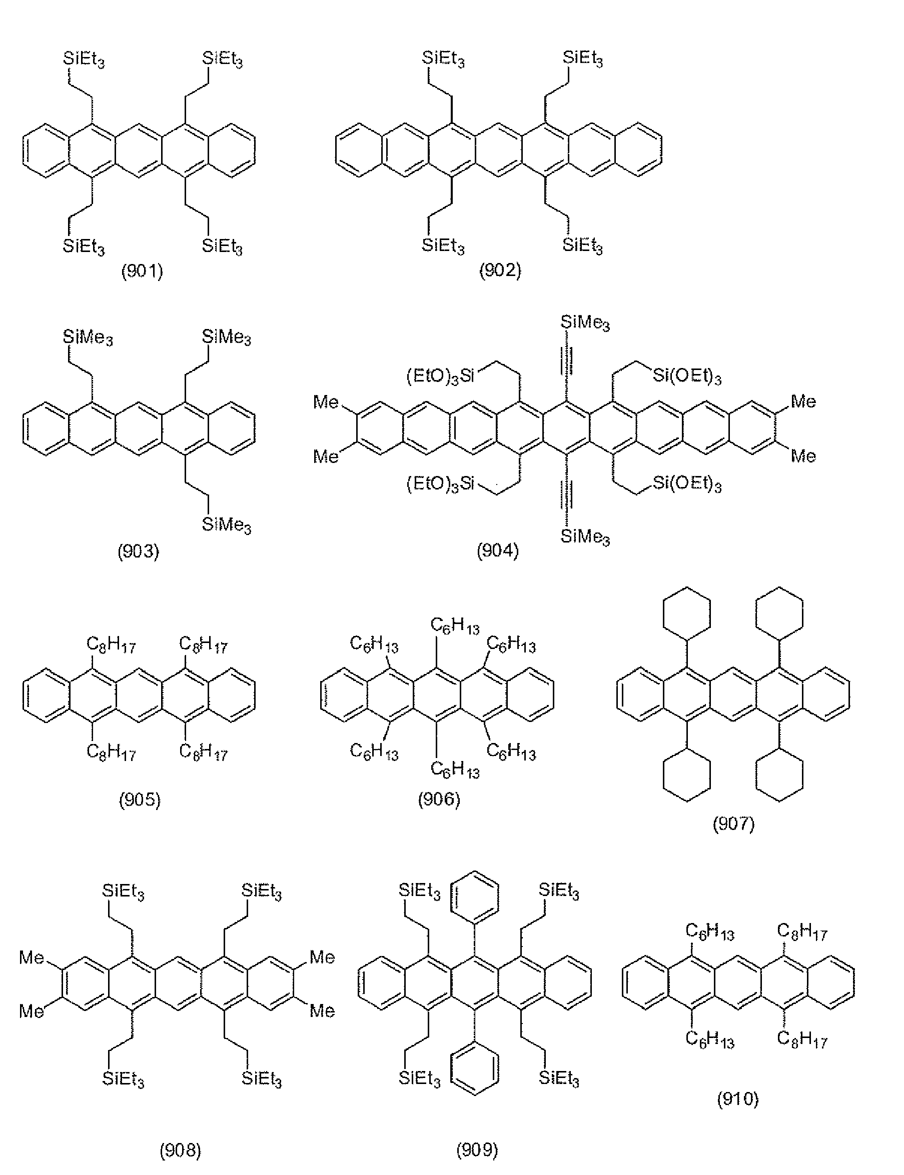

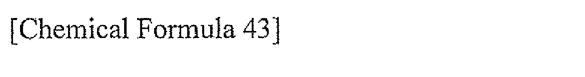

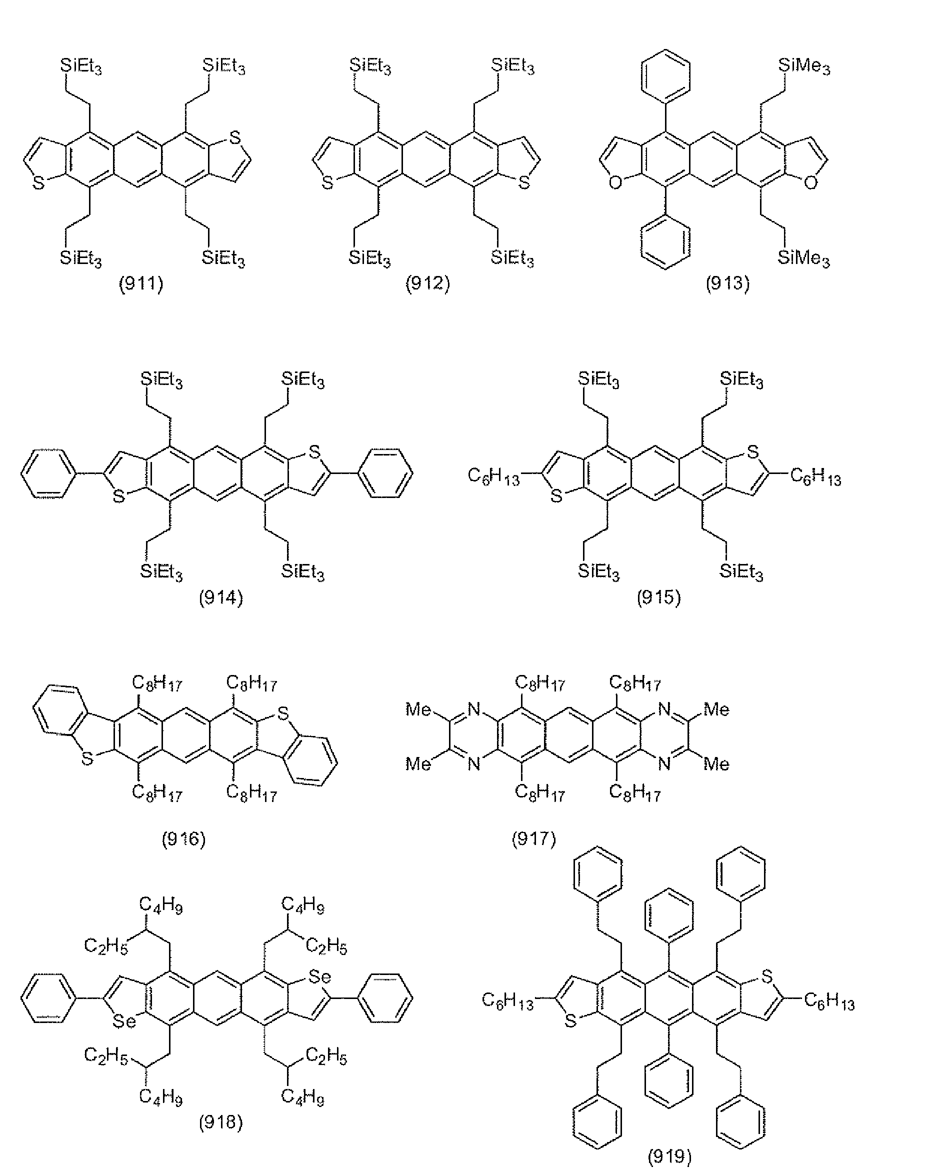

### [Organic thin-film]

An organic thin-film according to a preferred embodiment will now be explained. The organic thin-film is in the shape of a film and comprises an aromatic compound of the embodiment described above. According to a separate embodiment, the organic thin-film is in the shape of a film and comprises a polyacene compound represented by formula (7) or (8) above. The organic thin-film can exhibit a high charge transport property whether it contains an aromatic compound or a polyacene compound represented by formula (7) or (8).

The preferred thickness of the organic thin-film will differ depending on the element to which the organic thin-film is to be applied, but it will usually be in the range of 1 nm-100 µm, preferably 2 nm-1000 nm, more preferably 5 nm-500 nm and even more preferably 20 nm-200 nm.

The organic thin-film may comprise a single type of aromatic compound or polyacene compound, or two or more types. It may also comprise both the aromatic compound and the polyacene compound. The organic thin-film may further comprise a low molecular compound or polymer compound with an electron transport property or hole transport property in addition to the aromatic compound or polyacene compound, to increase the electron transport property or hole transport property. When the organic thin-film comprises a component other than the aromatic compound and polyacene compound, the aromatic compound or polyacene compound is preferably contained at 30 wt% or greater and more preferably 50 wt% or greater. If the content of the aromatic compound or polyacene compound is less than 30 wt%, it will tend to be difficult to form a thin-film or it may be difficult to obtain satisfactory charge mobility.

Examples of compounds with a hole transport property include pyrazoline derivatives, arylamine derivatives, stilbene derivatives, triphenyldiamine derivatives, oligothiophene and its derivatives, polyvinylcarbazole and its derivatives, polysilane and its derivatives, polysiloxane derivatives with aromatic amines on the side chains or main chain, polyaniline and its derivatives, polythiophene and its derivatives, polypyrrole and its derivatives, polyphenylenevinylene and its derivatives, and polythienylenevinylene and its derivatives.

Examples of compounds with an electron transport property include oxadiazole derivatives, quinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, polyfluorene and its derivatives, and C₆₀ or other fullerenes and their derivatives.

For improved physical properties, the organic thin-film may further comprise additional components. Such other components include charge generating materials. If the organic thin-film includes a charge generating material, the thin-film will absorb light to generate an electrical charge, and will be suitable for purposes such as optical sensors that require charge generation by light absorption.

Charge generating materials include azo compounds and their derivatives, diazo compounds and their derivatives, ametallic phthalocyanine compounds and their derivatives, metallic phthalocyanine compounds and their derivatives, perylene compounds and their derivatives, polycyclic quinone-based compounds and their derivatives, squarylium compounds and their derivatives, azulenium compounds and their derivatives, thiapyrilium compounds and their derivatives, and C₆₀ or other fullerenes and their derivatives.

The organic thin-film may also additionally contain materials necessary for exhibiting various functions. Examples thereof include sensitizing agents to enhance the function or generating charge by light absorption, stabilizers to increase stability, and UV absorbers for absorption of UV light.

The organic thin-film may also contain high molecular compound materials as high molecular binders, from the viewpoint of increasing the mechanical strength. Preferred as such high molecular binders are ones that do not excessively reduce the charge transport property, and those that do not excessively absorb visible light.

High molecular binders include poly(N-vinylcarbazole), polyaniline and its derivatives, polythiophene and its derivatives, poly(p-phenylenevinylene) and its derivatives, poly(2,5-thienylenevinylene) and its derivatives, polycarbonates, polyacrylates, polymethyl acrylates, polymethyl methacrylates, polystyrenes, polyvinyl chlorides, polysiloxanes and the like.

The organic thin-film may be produced by the following method, for example.

Specifically, the organic thin-film may be formed by a method in which a solution of the aromatic compound or the polyacene compound, and other components mentioned above as necessary, in a solvent, is coated onto a prescribed base material and the solvent is then volatilized off for removal (coating method). Since either the aromatic compound or polyacene compound has a structure in which an alkyl group is bonded to an interior benzene ring, it has excellent solubility in solvents and is advantageous for forming a homogeneous thin-film over a large area by such a coating method. When the aromatic compound or polyacene compound has a sublimating property, the organic thin-film may be formed by a method such as vacuum vapor deposition.

The solvent is preferably one that can dissolve or evenly disperse the aromatic compound or polyacene compound and the other components. Examples of such solvents include aromatic hydrocarbon-based solvents such as toluene, xylene, mesitylene, tetralin, decalin and n-butylbenzene, halogenated saturated hydrocarbon-based solvents such as carbon tetrachloride, chloroform, dichloromethane, dichloroethane, chlorobutane, bromobutane, chloropentane, bromopentane, chlorohexane, bromohexane, chlorocyclohexane and bromocyclohexane, halogenated aromatic hydrocarbon-based solvents such as chlorobenzene, dichlorobenzene and trichlorobenzene, and ether-based solvents such as tetrahydrofuran and tetrahydropyran. The aromatic compound or polyacene compound preferably dissolved to at least 0.1 wt% in the solvent.

The method for coating the solution may be spin coating, casting, microgravure coating, gravure coating, bar coating, roll coating, wire bar coating, dip coating, spray coating, screen printing, flexographic printing, offset printing, ink jet printing or dispenser printing. Particularly preferred are spin coating, flexographic printing, ink jet printing and dispenser printing.

The organic thin-film may be further subjected to a step of orienting the aromatic compound or polyacene compound in the organic thin-film, depending on the purpose of use. Such orientation can align the aromatic compound or polyacene compound in a prescribed direction in the organic thin-film, to further increase the charge transport property of the organic thin-film.

The method of orientating the organic thin-film may be a method commonly employed for orientation of liquid crystals and the like. Specifically, rubbing, photoorientation, shearing (shear stress application) and pull-up coating methods are preferred because they are convenient and useful, and rubbing and shearing are more preferred.

### [Organic thin-film transistor]

The organic thin-film of the embodiment described above has excellent charge (electron or hole) transport properties because it comprises an aromatic compound or a polyacene compound according to this embodiment. The organic thin-film can therefore efficiently transport electrons or holes introduced from an electrode or the like, or electrical charge generated by photoabsorption, thus allowing application of the organic thin-film in transistors and the like. An organic thin-film transistor according to a preferred embodiment will now be explained. The organic thin-film transistor may have a structure comprising a source electrode and drain electrode, an organic semiconductor layer (active layer) containing an aromatic compound or polyacene compound according to the aforementioned embodiment which acts as a current channel between them, and a gate electrode that controls the level of current flowing through the current channel, and the transistor may be a field-effect type or static induction type, for example.

A field-effect organic thin-film transistor preferably comprises a source electrode and drain electrode, an organic semiconductor layer (active layer) containing an aromatic compound or polyacene compound according to the embodiment which acts as a current channel between them, a gate electrode that controls the level of current flowing through the current channel, and an insulating layer situated between the active layer and the gate electrode. Most preferably, the source electrode and drain electrode are provided in contact with the organic semiconductor layer (active layer) containing the aromatic compound or polyacene compound of the embodiment, and the gate electrode is provided sandwiching the insulating layer which is in contact with the organic semiconductor layer.

On the other hand, a static induction-type organic thin-film transistor preferably comprises a source electrode and drain electrode, an organic semiconductor layer (active layer) containing an aromatic compound or polyacene compound according to the embodiment which acts as a current channel between them and a gate electrode that controls the level of current flowing through the current channel, with the gate electrode formed in the organic semiconductor layer. Most preferably, the source electrode, drain electrode and the gate electrode formed in the organic semiconductor layer are provided in contact with the organic semiconductor layer containing the aromatic compound or polyacene compound of the embodiment. The structure of the gate electrode may be any one that forms a current channel for flow from the source electrode to the drain electrode, and that allows the level of current flowing through the current channel to be controlled by the voltage applied to the gate electrode, it may be a combshaped electrode.

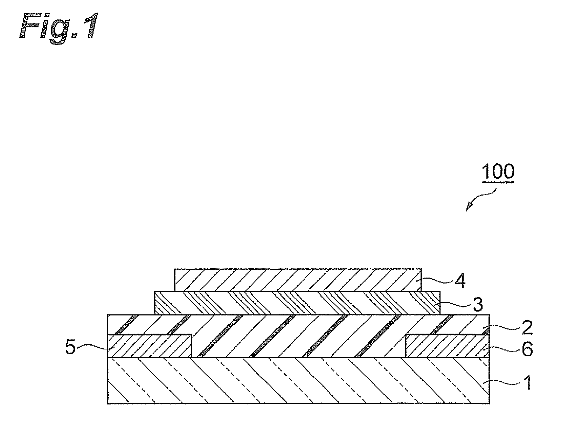

Fig. 1 is a schematic cross-sectional view of an organic thin--film transistor (field-effect organic thin-film transistor) according to a first embodiment. The organic thin-film transistor 100 shown in Fig. 1 comprises a substrate 1, a source electrode 5 and drain electrode 6 formed at a fixed spacing on the substrate 1 an active layer 2 formed on the substrate 1 covering the source electrode 5 and drain electrode 6, an insulating layer 3 formed on the active layer 2, and a gate electrode 4 formed on the insulating layer 3 covering the region of the insulating layer 3 between the source electrode 5 and drain electrode 6.

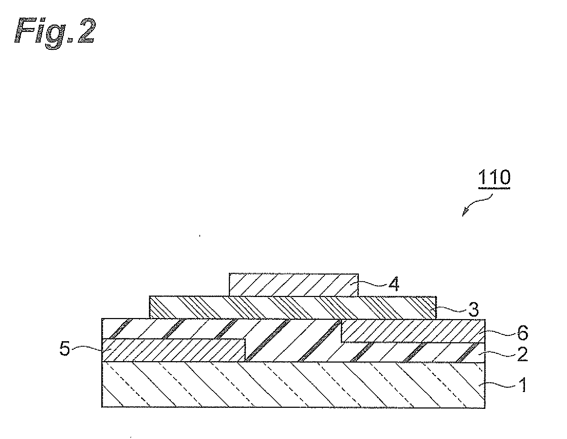

Fig. 2 is a schematic cross-sectional view of an organic thin-film transistor (field-effect organic thin-film transistor) according to a second embodiment. The organic thin-film transistor 110 shown in Fig. 2 comprises a substrate 1, a source electrode 5 formed on the substrate 1, an active layer 2 formed on the substrate 1 covering the source electrode 5, a drain electrode 6 formed on the active layer 2 at a prescribed spacing from the source electrode 5, an insulating layer 3 formed on the active layer 2 and drain electrode 6, and a gate electrode 4 formed on the insulating layer 3 covering the region of the insulating layer 3 between the source electrode 5 and drain electrode 6.

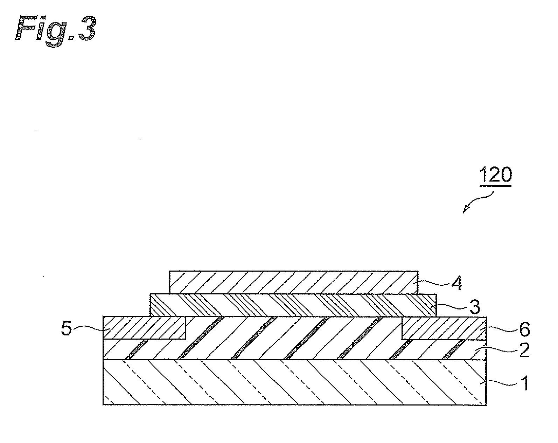

Fig. 3 is a schematic cross-sectional view of an organic thin-film transistor (field-effect organic thin-film transistor) according to a third embodiment. The organic thin-film transistor 120 shown in Fig. 3 comprises a substrate 1, an active layer 2 formed on the substrate 1, a source electrode 5 and drain electrode 6 formed at a prescribed spacing on the active layer 2, an insulating layer 3 formed on the active layer 2 covering the source electrode 5 and drain electrode 6, and a gate electrode 4 formed on the insulating layer 3, covering a portion of the region of the insulating layer 3 under which the source electrode 5 is formed and a portion of the region of the insulating layer 3 under which the drain electrode 6 is formed.

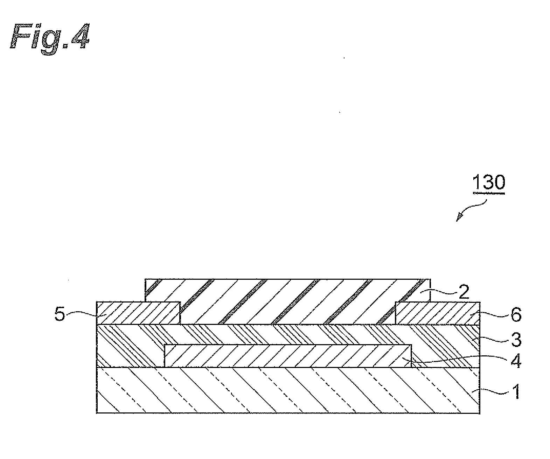

Fig. 4 is a schematic cross-sectional view of an organic thin-film transistor (field-effect organic thin-film transistor) according to a fourth embodiment. The organic thin-film transistor 130 shown in Fig. 4 comprises a substrate 1, a gate electrode 4 formed on the substrate 1, an insulating layer 3 formed on the substrate 1 covering the gate electrode 4, a source electrode 5 and drain electrode 6 formed at a prescribed spacing on the insulating layer 3 covering portions of the region of the insulating layer 3 under which the gate electrode 4 is formed, and an active layer 2 formed on the insulating layer 3 covering portions of the source electrode 5 and drain electrode 6.

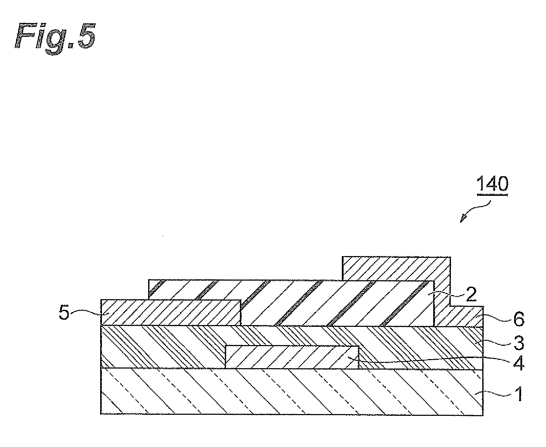

Fig. 5 is a schematic cross-sectional view of an organic thin-film transistor (field-effect organic thin-film transistor) according to a fifth embodiment. The organic thin-film transistor 140 shown in Fig. 5 comprises a substrate 1, a gate electrode 4 formed on the substrate 1, an insulating layer 3 formed on the substrate 1 covering the gate electrode 4, a source electrode 5 formed on the insulating layer 3 covering a portion of the region of the insulating layer 3 under which the gate electrode 4 is formed, an active layer 2 formed on the insulating layer 3 covering a portion of the source electrode 5, and a drain electrode 6 formed on the insulating layer 3 at a prescribed spacing from the source electrode 5 and covering a portion of the region of the active layer 2 under which the gate electrode 4 is formed.

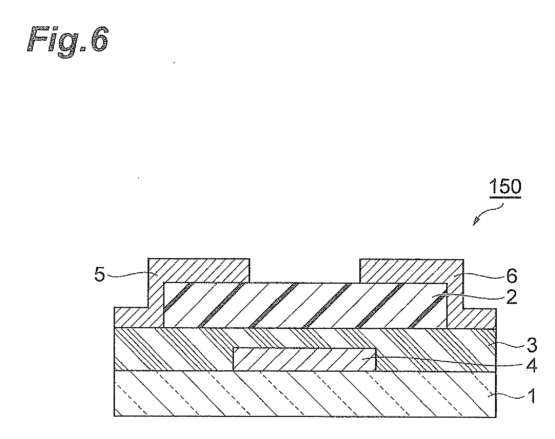

Fig. 6 is a schematic cross-sectional view of an organic thin-film transistor (field-effect organic thin-film transistor) according to a sixth embodiment. The organic thin-film transistor 150 shown in Fig. 6 comprises a substrate 1, a gate electrode 4 formed on the substrate 1, an insulating layer 3 formed on the substrate 1 covering the gate electrode 4, an active layer 2 formed covering the region of the insulating layer 3 under which the gate electrode 4 is formed, a source electrode 5 formed on the insulating layer 3 covering a portion of the region of the active layer 2 under which the gate electrode 4 is formed, and a drain electrode 6 formed on the insulating layer 3 at a prescribed spacing from the source electrode 5 and covering a portion of the region of the active layer 2 under which the gate electrode 4 is formed.

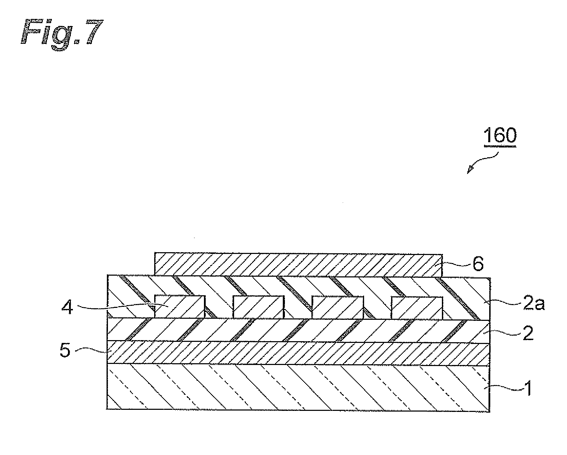

Fig. 7 is a schematic cross-sectional view of an organic thin-film transistor (static induction organic thin-film transistor) according to a seventh embodiment. The organic thin-film transistor 160 shown in Fig. 7 comprises a substrate 1, a source electrode 5 formed on the substrate 1, an active layer 2 formed on the source electrode 5, a plurality of gate electrodes 4 formed at prescribed spacings on the active layer 2, an active layer 2a formed on the active layer 2 covering all of the gate electrodes 4, (the material composing the active layer 2a may be the same as or different from that of the active layer 2) and a drain electrode 6 formed on the active layer 2a.

In the organic thin-film transistors of the first to seventh embodiments, the active layer 2 and/or the active layer 2a contains an aromatic compound or polyacene compound according to the aforementioned embodiment and forms a current channel between the source electrode 5 and drain electrode 6. The gate electrode 4 controls the level of current flowing through the current channel of the active layer 2 and/or active layer 2a by application of voltage.

This type of field-effect organic thin-film transistor can be manufactured by a publicly known process, such as the process described in Japanese Unexamined Patent Application Publication HEI No. 5-110069, for example. A static induction-type organic thin-film transistor can be manufactured by a publicly known process, such as the process described in Japanese Unexamined Patent Application Publication No. 2004-006476, for example.

The substrate 1 may be any one that does not impair the characteristics of the organic thin-film transistor, and a glass panel, flexible film substrate or plastic panel may be used.

Since organic solvent-soluble compounds are highly advantageous and preferred in forming the active layer 2, the method for producing an organic thin-film of the invention described above may be used to form organic thin-films composed of the active layer 2.

The insulating layer 3 in contact with the active layer 2 may be any material with high electrical insulating properties, and any publicly known one may be used. As examples there may be mentioned SiOx, SiNx, Ta₂O₅,polyimide, polyvinyl alcohol, polyvinylphenol, organic glass and photoresists. From the viewpoint of low voltage, a material with high permittivity is preferred.

When the active layer 2 is to be formed on the insulating layer 3, the active layer 2 may be formed after surface modification by treatment of the surface of the insulating layer 3 with a surface treatment agent such as a silane coupling agent in order to improve the interfacial properties between the insulating layer 3 and active layer 2. Surface treatment agents include long-chain alkylchlorosilanes, long-chain alkylalkoxysilanes, fluorinated alkylchlorosilanes, fluorinated alkylalkoxysilanes and silylamine compounds such as hexamethyldisilazane. Before treatment with the surface treatment agent, the insulating layer surface may be pre-treated by ozone UV or O₂ plasma.

After the organic thin-flm transistor has been fabricated, a protecting film is preferably formed on the organic thin-film transistor to protect the element. This will help prevent reduction in the characteristics of the organic thin-film transistor when the organic thin-film transistor has been blocked from air. A protecting film can also minimize adverse effects from the step of forming an operating display device on the organic thin-film transistor.

The method of forming the protecting film may involve covering with a UV curing resin, thermosetting resin, inorganic SiONₓ film or the like. For effective shielding from air, the steps after fabrication of the organic thin-flm transistor and before formation of the protecting film are preferably carried out without exposure to air (for example, in a dry nitrogen atmosphere or in a vacuum).

### Examples

The present invention will now be explained in greater detail through the following examples, with the understanding that these examples are in no way limitative on the invention.

### (Example 1: Synthesis of 5,7,12,14-tetrakis(2-(triethylsilyl)ethyl)pentacene-6,13-dione)

A 10 mL Schlenk tube and a Teflon^{R}-coated magnetic stirrer were placed in a drying oven and heated. After adequate heating, they were removed from the drying oven and the magnetic stirrer was placed in the Schlenk tube. The interior of the Schlenk tube was then exchanged with reduced-pressure nitrogen. After the Schlenk tube was cooled to room temperature, pentacene-6,13-dione (0.5 mmol, 154.1 mg) and a RuH₂(CO)(PPh₃)₃ complex (0.10 mmol, 91.9 mg) were added. After then exchanging the interior of the Schlenk tube with reduced-pressure nitrogen, toluene (1.0 mL) and triethylvinylsilane (5.0 mmol, 920 mL) were added.

The Schlenk tube was subsequently heated in an oil bath (145°C) and the interior mixture was reacted for 50 hours, after which the product was isolated. Isolation of the product was accomplished by purification using gel permeation chromatography (eluent = CHCl₃). As a result, 5,7,12,14-tetrakis(2-(triethylsilyl)ethyl)pentacene-6,13-dione was obtained at a 56% yield. The ¹H-NMR, ¹³C-NMR, IR and APCI-HRMS measurement results and the melting point for the obtained product were as follows.

¹H NMR (CDCl₃):δ 0.749 (q, 24H, *J* = 7.8 Hz, SiC*H*₂CH₃), 1.045-1.115 (m, 44H,CH₃ and ArCH₂C*H*₂Si), 3.30-3.50 (m, 8H,ArC*H*₂), 7.632 (dd, 4H, *J* = 3.4,6.3 Hz, Ar*H*), 8.206 (dd, 4H, *J* = 3.4,6.3 Hz, Ar*H*)
¹³C NMR CDCl₃):δ 3.424, 7.648, 15.259, 23.297, 126.133, 127.637, 131.467, 133.423, 141.231, 192.583
IR (KBr):3081 w, 2952 s, 2908 s, 2874 s, 2805 w, 2730 w, 1672 s, 1613 w, 1566 w, 1507 w, 1457 w, 1438 w, 1415 w, 1391 m, 1358 w, 1341 w, 1312 w, 1278 m, 1226 m, 1179 w, 1169 w, 1144 w, 1098 m, 1046 w, 1005 m, 833 w, 798 w, 771 m, 756 s, 735 s, 562 w cm⁻¹
APCI-HRMS (+): Calculated for C₅₄H₈₅O₂Si₄ (M+H)⁺ 877.56266, Found: 877.56130.
mp 1 71-171 .5°C

Next, 13.5 mg of 5,7, 12, 14-tetrakis(2-(triethylsilyl)ethyl)pentacene-6,13-dione was added to 51.6 mg of hexane at 22°C, and allowed to completely dissolve. The solubility in hexane was 26 wt%.

The reaction produced in this example is illustrated by the following reaction scheme.

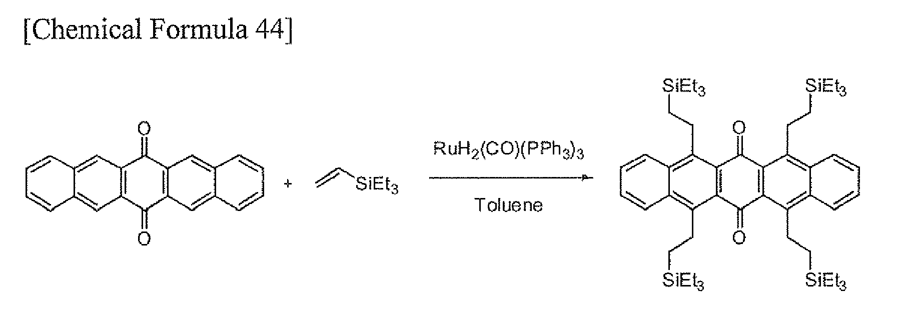

### (Example 2: Synthesis of 5,7,12, 1 4-tetrakis(2-(triethylsilyl)ethyl)pentacene)

A 10 mL Schlenk tube and a Teflon^{R}-coated magnetic stirrer were placed in a drying oven and heated. After adequate heating, they were removed from the drying oven and the magnetic stirrer was placed in the Schlenk tube. Next, the Schlenk tube was connected to a reduced pressure/nitrogen line and the entire reactor was exchanged with nitrogen. The reactor was allowed to cool to room temperature, and then 5,7,12,14-tetrakis(2-(triethylsilyl)ethyl)pentacene-6,13-dione (0.95 mmol, 43.9 mg) was added. The entire reactor was then exchanged with reduced-pressure nitrogen, after which acetic acid (1.0 mL) and iodic acid (0.5 mmol) were added.

The Schlenk tube was subsequently heated in an oil bath (140°C) and the interior mixture was reacted for 3 days. The reaction mixture was concentrated to obtain the target 5,7,12,14-tetrakis(2-(triethylsilyl)ethyl)pentacene.

The reaction produced in this example is illustrated by the following reaction scheme.

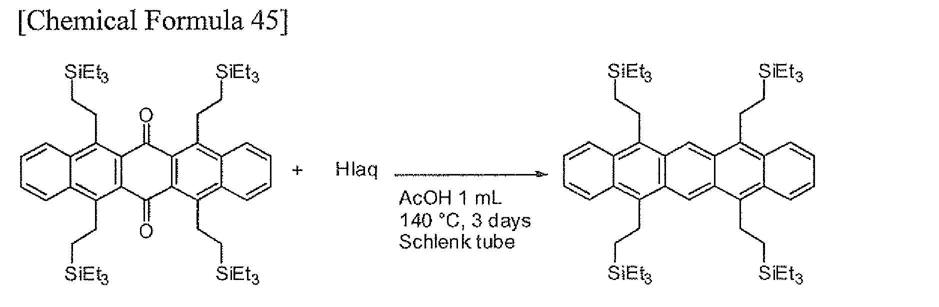

### (Example 3: Synthesis of 5,7,12,14-tetrakis(2-(triethylsilyl)ethyl)pentacene)

A 5 mL two-necked flask, a reflux condenser, a blowing tube and a Teflon^{R}-coated magnetic stirrer were placed in a drying oven and heated. After adequate heating, they were removed from the drying oven and the magnetic stirrer was placed in the two-necked flask. The reflux condenser and blowing tube were then mounted on the two-necked flask. The blowing tube was connected to a pressure reduction/nitrogen line and the entire reactor was exchanged with nitrogen. The reactor was allowed to cool to room temperature, and then 5,7,12,14-tetrakis(2-(triethylsilyl)ethyl)pentacene-6,13-dione (0.1 mmol, 88.0 mg) was added. After adding *tert*-butyl methyl ether (TBME)(2.0 mL), the mixture was cooled to 0°C in an ice bath. There was then added LiAlH₄ (0.4 mmol) to the solution. After removal from the ice bath, the reaction mixture was heated in an oil bath and reacted for 1 hour under a nitrogen atmosphere.

The reaction mixture was then cooled to 0°C in an ice bath. After adding 1 mol/L hydrochloric acid (1 mL) to the reaction mixture, the reaction mixture was heated in an oil bath and reacted for 3 hours under a nitrogen atmosphere. The reaction mixture was transferred to a 10 mL Schlenk tube, and then nitrogen-exchanged dichloromethane and nitrogen-exchanged distilled water were added thereto and the product was extracted. The organic layer was concentrated by evaporation to obtain the target 5,7,12,14-tetrakis(2-(triethylsilyl)ethyl)pentacene at 95% yield (80.6 mg). The ¹H-NMR and APCI-MS measurement results for the obtained product were as follows.

¹H NMR (acetane-*d₆*):δ 0.80 (m, 32H, Si*CH₂*CH₃ and ArCH₂*CH₂*Si), 1.09 (t, J = 8.1 Hz, 36H,CH₃), 3.92-4.41 (m, 8H, ArCH₂), 7.45-7.49 (m, 4H, ArH), 8.32-8.36 (m, 4H, ArH), 9.64 (s, 2H, ArH).
APCI-MS (+): m/z = 847 (M+1,68), 846 (M, 87), 845 (M-1,100) cf. Chemical Formula: C₅₄H₈₆O₂Si₄,
Exact Mass: 846.5807,
Molecular Weight: 847.6026

The reaction produced in this example is illustrated by the following reaction scheme.

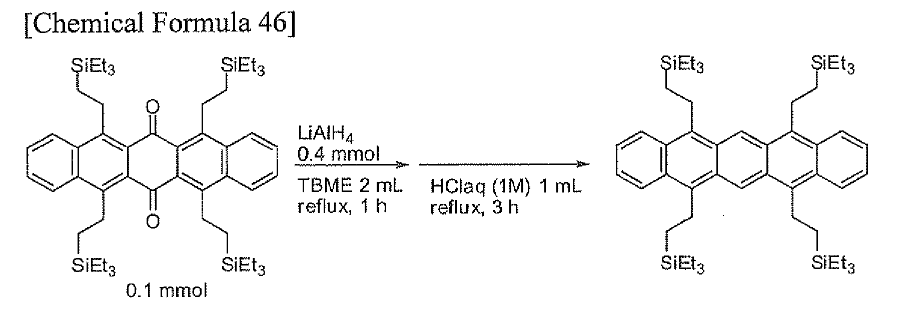

### (Example 4: Evaluation of field-effect type organic thin-film transistor)

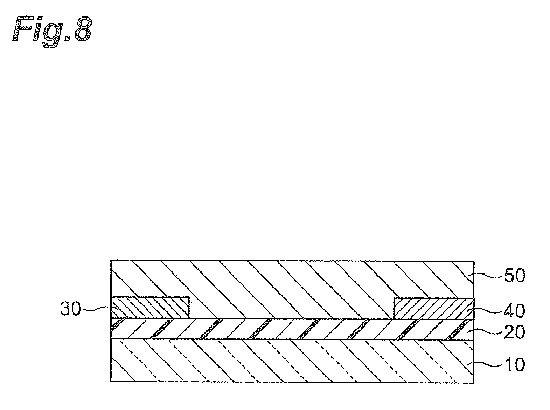

The transistor property of the 5,7,12,14-tetrakis(2-(triethylsilyl)ethyl)pentacene-6,13-dione was measured by fabricating a field-effect type organic thin-film transistor. Fig. 8 shows a schematic cross-sectional view of the fabricated organic thin-film transistor. The surface of an n-type silicon substrate 10 doped to a high concentration, as a gate electrode, was thermally oxidized to form a 300 nm silicon oxide film (hereunder referred to as "thermal oxidation film") 20. Next, a source electrode 30 and drain electrode 40 with a channel length of 20 µm and a channel width of 2 mm were formed on a thermal oxidation film 20 (vapor deposition of chromium and gold in that order from the thermal oxidation film 20 side) by photolithography. The board obtained in this manner was thoroughly washed and then hexamethylenedisilazane was used for silane treatment of the board surface by spin coating.

Next, a mixture of 5,7,12,14-tetrakis(2-(triethylsilyl)ethyl)pentacene-6,13-dione and a polymer compound represented by the following formula: 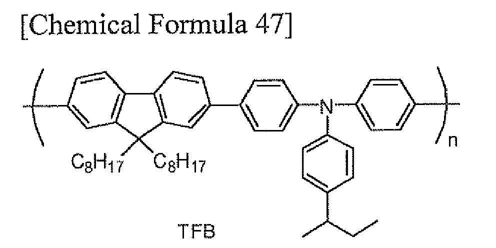 (TFB, number-average molecular weight: Mn = 6.4 × 10⁴, weight-average molecular weight: Mw = 1.6 × 10⁵) (weight ratio = 1:1) was dissolved in chloroform to prepare a 0.5 wt% solution, which was filtered with a membrane filter and then spin coated onto a surface-treated substrate to form an approximately 80 nm organic semiconductor layer 50.

When the transistor property was measured while varying the gate voltage Vg to the field-effect type organic thin-film transistor obtained in this manner between 0 V and -60 V, and with the voltage Vsd between the source-drain at -50 V, a carrier mobility of 5.8 × 10⁻⁴ cm²/Vs was exhibited.

### (Example 5: Evaluation of field-effect type organic thin-film transistor)

The transistor property of the 5,7,12,14-tetrakis(2-(triethylsilyl)ethyl)pentacene was measured by fabricating a field-effect type organic thin-film transistor. Fig. 8 shows a schematic cross-sectional view of the fabricated organic thin-film transistor. The surface of an n-type silicon substrate 10 doped to a high concentration, as a gate electrode, was thermally oxidized to form a 300 nm silicon oxide film (hereunder referred to as "thermal oxidation film") 20. Next, a comb source electrode 30 and comb drain electrode 40 with a channel length of 5 µm and a channel width of 38 mm were formed on a thermal oxidation film 20 (vapor deposition of gold) by photolithography.

Next, the 5,7,12,14-tetrakis(2-(triethylsilyl)ethyl)pentacene was dissolved in chloroform to prepare a 1 wt% solution, which was spin coated onto a surface-treated substrate (1500 rpm, 60 sec) to form an organic semiconductor layer 50. It was then heated at 100°C for 30 minutes in a vacuum.

When the transistor properties were measured while varying the gate voltage Vg to the field-effect type organic thin-film transistor obtained in this manner between 0 V and -80 V, and with the voltage Vsd between the source-drain at -50 V, a carrier mobility of 4.8 × 10⁻⁶ cm₂/Vs was exhibited.

### (Example 6: Synthesis of 4,6,10,12-tetrakis(2-(triethylsilyl)ethyl)anthra[2,3-b:6,7-b']dithiophene-5,11-dione and 4,6,10,12-tetrakis(2-(triethylsilyl)ethyl)anthra[2,3-b:7,6-b']dithiophene-5,11-done)

A 10 mL Schlenk tube and a Tefion^{R}-coated magnetic stirrer were placed in a drying oven and heated. After adequate heating, they were removed from the drying oven and the magnetic stirrer was placed in the Schlenk tube. The interior of the Schlenk tube was then exchanged with reduced-pressure nitrogen. The Schlenk tube was cooled to room temperature, and a 1:1 mixture of anthra[2,3-b:6,7-b']dithiophene-5,11-dione and anthra[2,3-b:7,6-b']dithiophene-5,11-dione (0.5 mmol, 154.1 mg) was added to a RuH₂(CO)(PPh₃)₃ complex (0.05 mmol, 45.9 mg). After then exchanging the interior of the Schlenk tube with reduced-pressure nitrogen, toluene (1.0 mL) and triethylvinylsilane (5.0 mmol, 920 mL) were added.

The Schlenk tube was subsequently heated in an oil bath (115°C) and the interior mixture was reacted for 48 hours, after which the product was isolated. Isolation of the product was accomplished by purification using gel permeation chromatography (eluent = CHCl₃). As a result there was obtained a 1:1 mixture of 4,6,10,12-tetrakis(2-(triethylsilyl)ethyl)anthra[2,3-b:6,7-b']dithiophene-5,11-done and 4,6,10, 12-tetrakis(2-(triethylsilyl)ethyl)anthra[2,3-b:7,6-b']dithiophene-5,11-dine, at a 28% yield. The ¹H-NMR and ¹³C-NMR measurement results for the obtained product were as follows.

¹H NMR (CDCl₃):δ 0.70-0.80 (m, 24H, SiCH₂CH₃), 1.04-1.15 (m, 44H,CH₃ and ArCH₂CH₂Si), 3.22-3.31 (m, 8H, ArCH₂), 7.54-7.50 (m, 2H, thiophene-H), 7.63-7.69 (m, 2H, thiophene-H)
¹³C NMR (CDCl₃):δ 3.37, 3.41, 7.63, 7.64, 13.24, 13.30, 14.93, 14.98, 25.42, 25.45, 27.93, 27.97, 123.94, 129.40, 129.79, 131.05, 131.38, 138.70, 138.88, 139.76, 139.93, 141.39,144.31, 144.44,190.20, 190.88, 191.57

Next, 9.3 mg of a 1:1 mixture of 4,6,10,1 1 2-tetrakis(2-(triethylsilyl)ethyl)anthra[2,3-b :6,7-b']dithiophene-5,11-dione and 4,6,10,12-tetrakis(2-(triethylsilyl)ethyl)anthra[2,3-b:7,6-b']dithiophene-5,11-dione was added to 180.1 mg of hexane at 26°C, and allowed to completely dissolve. The solubility in hexane was 5 wt%.

The reaction produced in this example is illustrated by the following reaction scheme.

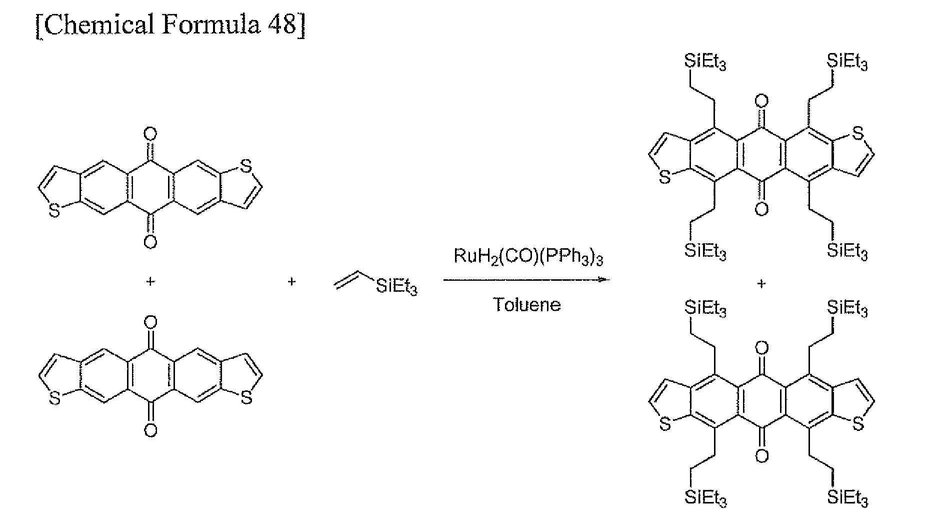

### (Example 7: Synthesis of 4,6,10,12-tetrakis(2-(triethylsilyl)ethyl)anthra[2,3-b:6,7-b']dithiophene and

4,6,10,12-tetrakis(2-(triethylsilyl)ethyl)anthra[2,3-b:7,6-b']dithiophene) A 5 mL two-necked flask, a reflux condenser, a blowing tube and a Teflon^{R}-coated magnetic stirrer were placed in a drying oven and heated. After adequate heating, they were removed from the drying oven and the magnetic stirrer was placed in the two-necked flask. The reflux condenser and blowing tube were then mounted on the two-necked flask. The blowing tube was connected to a pressure reduction/nitrogen line and the entire reactor was exchanged with nitrogen. After cooling the reactor to room temperature, a 1: mixture of 4,6,10,12-tetrakis(2-(triethylsilyl)ethyl)anthra[2,3-b:6,7-b']dithiophenc-5,11-dione and 4,6,10,12-tetrakis(2-(triethylsilyl)ethyl)anthra[2,3-b:7,6-b']dithiophene-5,11-done (0.05 mmol, 43.1 mg) was added. After adding tert-butyl methyl ether (TBME)(1.0 mL), the mixture was cooled to 0°C in an ice bath. There was then added LiAlH₄ (0.2 mmol) to the solution. After removal from the ice bath, the reaction mixture was heated in an oil bath and reacted for 1 hour under a nitrogen atmosphere.

The reaction mixture was then cooled to 0°C in an ice bath. After adding 1 mol/L hydrochloric acid (0.5 mL) to the reaction mixture, it was heated in an oil bath and reacted for 3 hours under a nitrogen atmosphere. The reaction mixture was transferred to a 10 mL Schlenk tube, and then nitrogen-exchanged dichloromethane and nitrogen-exchanged distilled water were added thereto and the product was extracted. The organic layer was concentrated by evaporation to obtain a 1:1 mixture of the target 4,6,10,12-tetrakis(2-(triethylsilyl)ethyl)anthra[2,3-b:6,7-b']dithiophene and 4,6,10,12-tetrakis(2-(triethylsilyl)ethyl)anthra[2,3-b:7,6-b']dithiophene at a 64% yield (26.6 mg). The ¹H-NMR measurement results for the obtained product were as follows.

¹H NMR (CDCl₃):δ 0.60-1.3 (m, SiCH₂CH₃ and SiCH₂), 3.45-3.50 (m, ArCH₂), 3.55-3.65 (m, ArCH₂), 7.40-7.52 (m, thiophene-H), 7.63-7.69 (m, thiophene-H), 8.99 (s, ArH), 9.11 (s, ArH), 9.23 (s, ArH)

The reaction produced in this example is illustrated by the following reaction scheme.

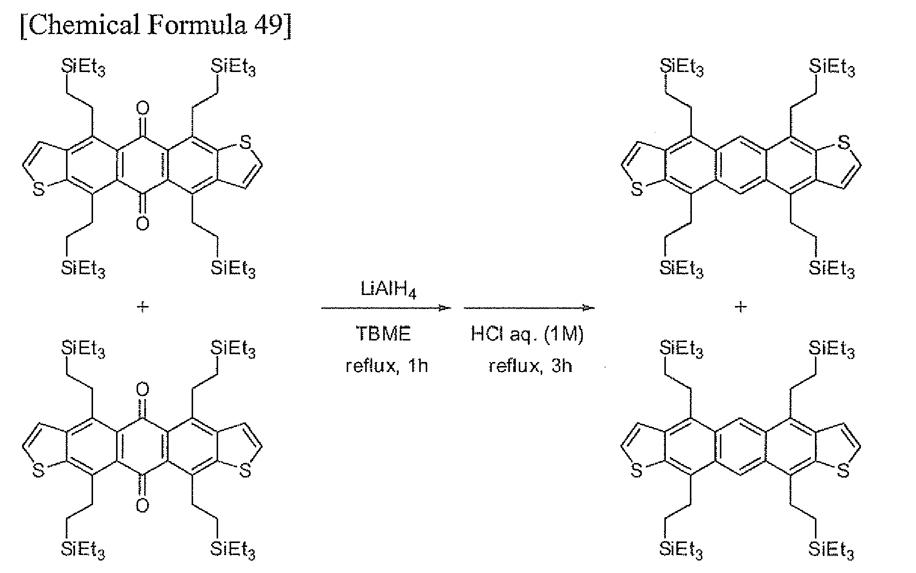

### Reference Signs List

1: Substrate, 2: active layer, 2a: active layer, 3: insulating layer, 4: gate electrode, 5: source electrode, 6: drain electrode, 10: gate electrode, 20: insulating layer (silicon oxide film, thermal oxidation film), 30: source electrode, 40: drain electrode, 50: organic semiconductor layer, 100: first embodiment of organic thin-film transistor, 110: second embodiment of organic thin-film transistor, 120: third embodiment of organic thin-film transistor, 130: fourth embodiment of organic thin-film transistor, 140: fifth embodiment of organic thin-film transistor, 150: sixth embodiment of organic thin-film transistor, 160: seventh embodiment of organic thin-film transistor.

## Claims

1. An aromatic compound represented by the following formula (1). 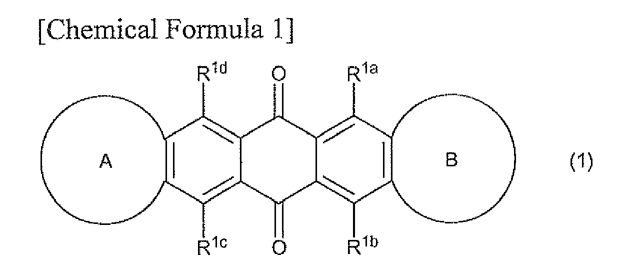 [In the formula, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2c} may be bonded together to form a ring. When more than one R^{2d}, R^{2e} or R^{2f} are present, the groups denoted by the same symbols may be the same groups or different groups.]

2. The aromatic compound according to claim 1, wherein ring A and ring B are each independently an optionally substituted benzene ring or an optionally substituted aromatic fused ring composed of 2-4 rings.

3. The aromatic compound according to claim 1 or 2, which is a compound represented by the following formula (2). 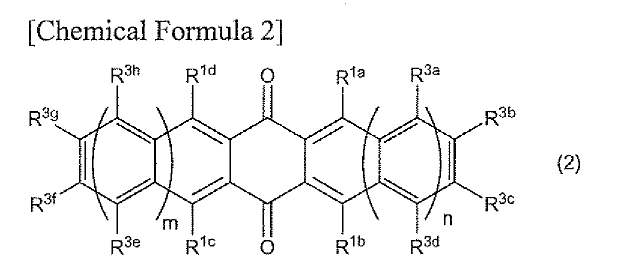 [In the formula, R^{1a} represents a group -CHR^{2a}-CHR^{2b}R^{2C}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. The denotations m and n each independently represent an integer of 1-3. R^{3a}, R^{3b}, R^{3c}, R^{3d}, R^{3e}, R^{3f}, R^{3g} and R^{3h} each independently represent hydrogen, optionally substituted alkyl, optionally substituted alkoxy or optionally substituted aryl. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups.]

4. The aromatic compound according to claim 3, wherein m = n.

5. The aromatic compound according to claim 4, wherein m = n = 1.

6. The aromatic compound according to any one of claims 1 to 5, wherein R^{1b,} R^{1c} and R^{1d} are each independently a group represented by -CHR^{2d}-CHR^{2e}R^{2f}_{.}

7. The aromatic compound according to claim 1, which is a compound represented by the following formula (3). 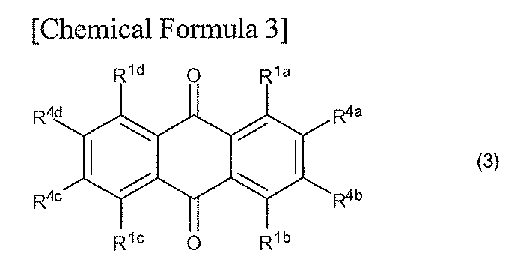 [In the formula, R^{1a} and R^{1c} are each a group represented by -CHR^{2a}-CHR^{2b}R^{2c} and are the same group, R^{1b} and R^{1d} are each an optionally substituted aryl group or a group represented by -CHR^{2d}-CHR^{2e}R^{2f} and are the same group, and R^{4a} and R^{4b}, and R^{4c} and R^{4d}, are bonded together to form ring B and ring A, in which R^{4a} and R^{4c} are the same group and R^{4b} and R^{4d} are the same group. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring.]

8. The aromatic compound according to claim 1, wherein ring A and ring B have a structure represented by the following formula (12). 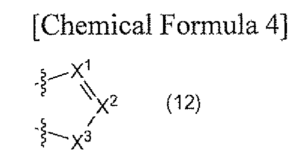 [In the formula, X¹ and X² each independently represent a carbon atom or nitrogen atom, and in the case or a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by -X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group.]

9. A method for producing an aromatic compound represented by the following formula (6), the method comprising a step of addition reaction of a compound represented by formula (5) with a compound represented by formula (4), in the presence of a transition metal complex. 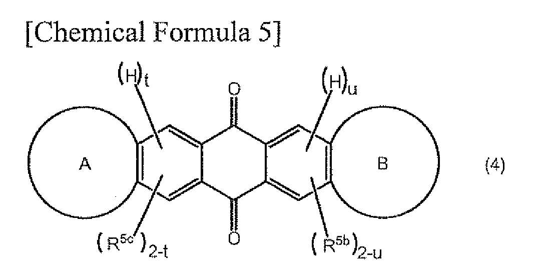 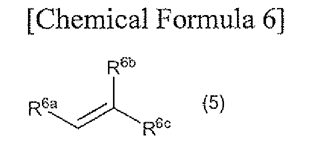 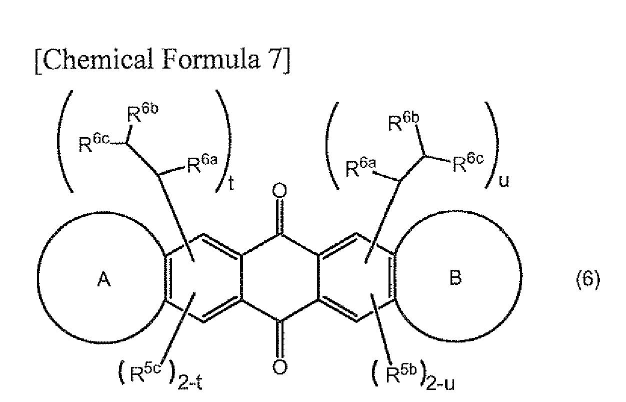 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{5b} and R^{5c} each independently represent hydrogen, optionally substituted aryl or a group -CHR^{8a}-CHR^{8b}R^{8c}. R^{6a}, R^{6b} and R^{6c} each independently represent hydrogen, optionally substituted alkyl, optionally substituted aryl or a substituted silyl group, and R^{6a} and R^{6b} may be bonded together to form a ring. R^{8a}, R^{8b} and R^{8c} each independently represent hydrogen, optionally substituted alkyl, optionally substituted aryl or a substituted silyl group, and R^{8a} and R^{8b} may be bonded together to form a ring. The denotations t and u each independently represent an integer of 0-2, and are values such that t+u ≥ 1 is satisfied. In the case of two R^{5c} groups, the two R^{5c} groups may be the same or different, and in the case of two R^{5b} groups the two R^{5b} groups may be the same or different.]

10. The method for producing an aromatic compound according to claim 9, wherein ring A and ring B are each independently an optionally substituted benzene ring or an optionally substituted aromatic fused ring composed of 2-4 rings.

11. The method for producing an aromatic compound according to claim 9 or 10, wherein at least two of R^{5b} and R^{5c} are not hydrogen atoms.

12. The method for producing an aromatic compound according to any one of claims 9 to 11, wherein the transition metal complex is a complex comprising a transition metal of groups 8-10 of the Periodic Table.

13. The method for producing an aromatic compound according to claim 12, wherein the transition metal complex is a ruthenium complex.

14. The method for producing an aromatic compound according to claim 13, wherein the transition metal complex is RuH₂(PPh₃)₄ or RuH₂(CO)(PPh₃)₃.

15. A polyacene compound represented by the following formula (15). 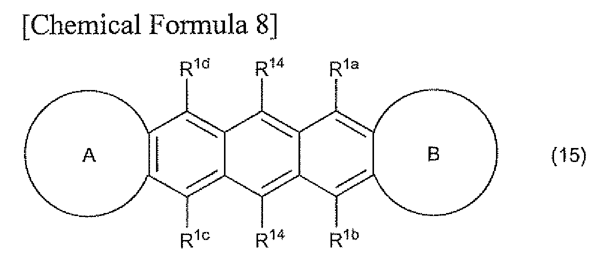 [In the formula, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups. R¹⁴ represents hydrogen or an optionally substituted alkyl, optionally substituted aryl, optionally substituted heteroaryl, optionally substituted alkenyl or optionally substituted alkynyl group. When multiple R¹⁴ groups are present, they may be the same or different.]

16. The polyacene compound according to claim 15, wherein R^{1b}, R^{1c} and R^{1d} are each independently a group represented by -CHR^{2d}-CHR^{2e}R^{2f}.

17. The polyacene compound according to claim 16, wherein R^{1a}, R^{1b} R^{1c} and R^{1d} are the same group, and all of the substituents of ring A and ring B are the same groups.

18. A polyacene compound represented by the following formula (13). 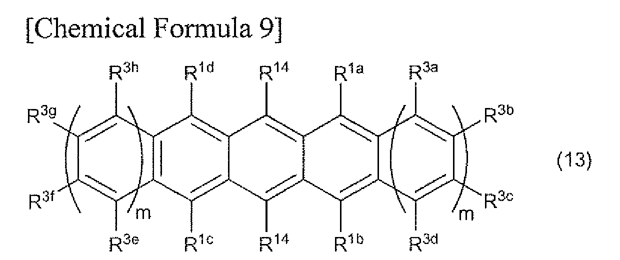 [In the formula, R^{1a} represents a group -CHR^{2a}-CHR^{2b}R^{2c}, and R^{1b}, R^{lc} and R^{1d} each independently represent hydrogen, optionally substituted aryl or a group -CHR^{2d}-CHR^{2c}R^{2f}. This is with the proviso that at least 2 of R^{1b}, R^{1c} and R^{1d} are not hydrogen. The denotation m represents an integer of 1-3_{.} R^{3a}, R^{3b}, R^{3c}, R^{3d}, R^{3e}, R^{3f}, R^{3g} and R^{3h} each independently represent hydrogen, optionally substituted alkyl, optionally substituted alkoxy or optionally substituted aryl. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2c} may be bonded together to form a ring. When more than one R^{2d}, R^{2e} or R^{2f} are present, the groups denoted by the same symbols may be the same groups or different groups. R¹⁴ represents hydrogen or an optionally substituted alkyl, optionally substituted aryl, optionally substituted heteroaryl, optionally substituted alkenyl or optionally substituted alkynyl group. When multiple R¹⁴ groups are present, they may be the same or different.]

19. The polyacene compound according to claim 18, wherein m = 1.

20. The polyacene compound according to claim 15, wherein ring A and ring B have a structure represented by the following formula (12). 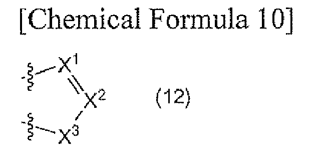 [In the formula, X¹ and X² each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group. X³ represents a nitrogen atom, oxygen atom, sulfur atom or selenium atom or a group represented by -X⁵=X⁶-, and in the case of a nitrogen atom, there may be bonded to the nitrogen atom: a hydrogen atom, an optionally substituted alkyl group, an optionally substituted alkoxy group or an optionally substituted aryl group. X⁵ and X⁶ each independently represent a carbon atom or nitrogen atom, and in the case of a carbon atom, there may be bonded to the carbon atom: a hydrogen atom, an optionally substituted alkyl group, an optionally substituted alkoxy group or an optionally substituted aryl group.]

21. The polyacene compound according to claim 20, wherein X³ is a nitrogen atom, oxygen atom, sulfur atom or selenium atom.

22. The polyacene compound according to claim 21, wherein X³ is a sulfur atom, and X¹ and X² are carbon atoms.

23. A method for producing a polyacene compound represented by formula (7), comprising a step of reducing an aromatic compound represented by formula (1). 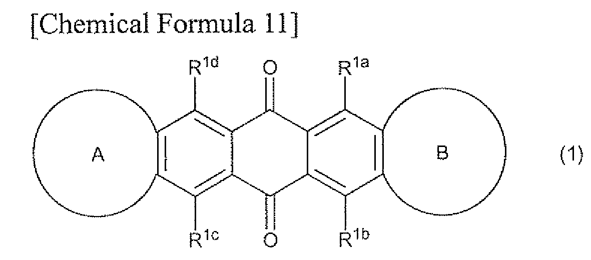 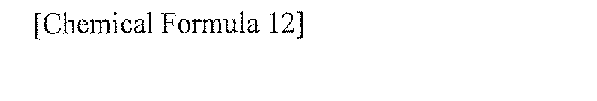 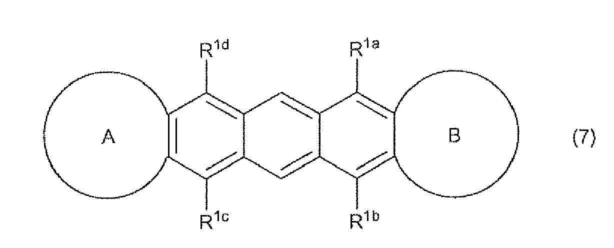 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d} R^{2e} or R^{2f} are present, the groups denoted by the same symbols may be the same groups or different groups.]

24. The method for producing a polyacene compound according to claim 23, wherein ring A and ring B are each independently an optionally substituted benzene ring or an optionally substituted aromatic fused ring composed of 2-4 rings.

25. A method for producing a polyacene compound represented by formula (8), the method comprising a step of reducing a diol compound obtained by reaction between an aromatic compound represented by formula (1) and an organometallic compound. 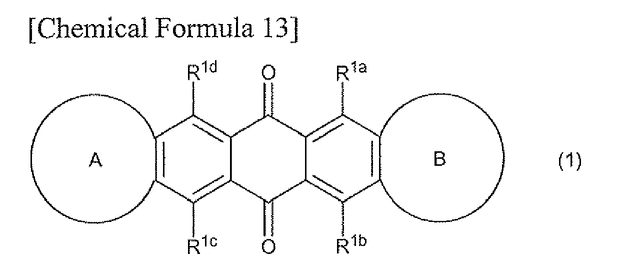 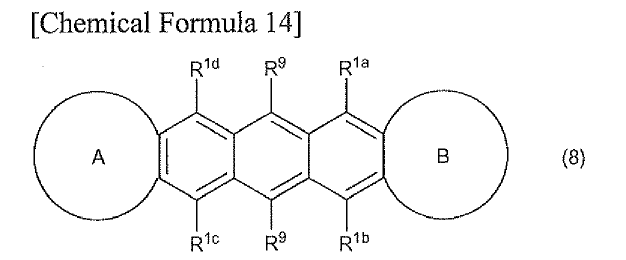 [In the formulas, ring A and ring B each independently represent an optionally substituted benzene ring, an optionally substituted aromatic fused ring composed of 2-4 rings, an optionally substituted heteroaromatic ring or an optionally substituted heterocyclic aromatic fused ring composed of 2-4 rings, R^{1a} represents a group -CHR^{2a}-CHR^{2b}R^{2c}, and R^{1b}, R^{1c} and R^{1d} each independently represent hydrogen, optionally substituted aryl or a group -CHR^{2d}-CHR^{2e}R^{2f}. This is with the proviso that at least 2 of R^{1b}, R^{1c} and R^{1d} are not hydrogen. R^{2a}, R^{2b} and R^{2c} each independently represent hydrogen, optionally substituted alkyl, optionally substituted aryl or a substituted silyl group, and R^{2a} and R^{2b} may be bonded together to form a ring. R^{2d}, R^{2e} and R^{2f} each independently represent hydrogen, optionally substituted alkyl, optionally substituted aryl or a substituted silyl group, and R^{2d} and R^{2e} may be bonded together to form a ring. When more than one R^{2d}, R^{2e} or R^{2f} are present, the groups denoted by the same symbols may be the same groups or different groups. R⁹ represents an optionally substituted alkyl, optionally substituted aryl, optionally substituted heteroaryl, optionally substituted alkenyl or optionally substituted alkynyl group. When multiple R⁹ groups are present, they may be the same or different.]

26. The method for producing the polyacene compound according to claim 25, wherein ring A and ring B are each independently an optionally substituted benzene ring or an optionally substituted aromatic fused ring composed of 2-4 rings.

27. An organic thin-film comprising the aromatic compound according to any one of claims 1 to 8.

28. An organic thin-film comprising the polyacene compound produced by the production method according to any one of claims 23 to 26.

29. An organic thin-film transistor comprising a source electrode and drain electrode, an organic semiconductor layer serving as a current channel between the electrodes and a gate electrode that controls the level of current flowing through the current channel, wherein the organic semiconductor layer comprises the organic thin-film according to claim 27 or 28.
